(19) **Europäisches Patentamt**
**European Patent Office**
**Office européen des brevets**

(11) **EP 4 091 172 B1**

(12) **EUROPEAN PATENT SPECIFICATION**

(45) Date of publication and mention
of the grant of the patent:
**04.03.2026 Bulletin 2026/10**

(21) Application number: **21701006.5**

(22) Date of filing: **15.01.2021**

(51) International Patent Classification (IPC):
***G16B 30/00*** *(2019.01)* ***G16B 40/20*** *(2019.01)*

(52) Cooperative Patent Classification (CPC):
**G16B 30/00; G16B 20/00; G16B 40/20;** Y02A 90/10

(86) International application number:
**PCT/IB2021/050275**

(87) International publication number:
**WO 2021/144742 (22.07.2021 Gazette 2021/29)**

(54) **ANCESTRY INFERENCE BASED ON CONVOLUTIONAL NEURAL NETWORK**

RÜCKSCHLÜSSE AUF DIE ABSTAMMUNG BASIEREND AUF EINEM GEFALTETEN
NEURONALEN NETZ

INFÉRENCE D'ASCENDANCE BASÉE SUR UN RÉSEAU NEURONAL CONVOLUTIONNEL

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **17.01.2020 US 202062962786 P**
**13.05.2020 US 202015931009**

(43) Date of publication of application:
**23.11.2022 Bulletin 2022/47**

(73) Proprietor: **Ancestry.com DNA, LLC
Lehi, UT 84043 (US)**

(72) Inventors:
• **MCMASTER-SCHRAIBER, Joshua Goodwin Jon
Lehi, Utah 84043 (US)**
• **SONG, Shiya
Lehi, Utah 84043 (US)**

• **WANG, Yong
Lehi, Utah 84043 (US)**

(74) Representative: **Meissner Bolte Nürnberg
Patentanwälte Rechtsanwälte
Partnerschaft mbB
Bankgasse 3
90402 Nürnberg (DE)**

(56) References cited:
**US-A1- 2019 114 219 US-A1- 2020 005 899**

• **GHAZALEH KHODABANDELOU ET AL:
"ABSTRACT", BIORXIV, 7 June 2019
(2019-06-07), XP055658573, Retrieved from the
Internet <URL:https://www.biorxiv.org/content/
10.1101/330308v1.full.pdf> DOI: 10.1101/330308**

**Description**

**CROSS-REFERENCE TO RELATED APPLICATIONS**

The present application is a continuation in part application of U.S. Application No.: 15/931,009, filed on May 13, 2020, which is a continuation application of U.S. Application No.: 16/567,957, filed on September 11, 2019, which claims the benefit of U.S. Provisional Patent Applications No. 62/729,840 filed on September 11, 2018, 62/743,448 filed on October 9, 2018, 62/752,523 filed on October 30, 2018, and 62/858,820 filed on June 7, 2019. The present application also claims the benefit of U.S. Provisional Patent Applications No. 62/962,786 filed on January 17, 2020.

**FIELD**

The disclosed embodiments relate to assigning labels to an input sample genotype. In particular, the disclosed embodiments relate to using a Convolutional Neural Network (CNN) to efficiently and accurately determine labels for the input sample genotype.

**BACKGROUND**

Although humans are, genetically speaking, almost entirely identical, small differences in human DNA are responsible for much of the variation between individuals. For example, a sequence variation at one position in DNA between individuals is known as a single-nucleotide polymorphism (SNP). Stretches of DNA inherited together from a single parent are referred to as haplotypes (e.g., one haplotype inherited from the mother and another haplotype inherited from the father).

A subset of the SNPs in an individual's genome may be detected with SNP genotyping. Through SNP genotyping, the pair of alleles for a SNP at a given location in each haplotype may be identified. For example, a genotype at a SNP locus may be identified as heterozygous (i.e., one allele of each type), homozygous (i.e., both alleles of a same type), or unknown. SNP genotyping identifies the pair of alleles for a given genotype, but does not identify which allele corresponds to which haplotype, i.e., SNP genotyping does not identify the homomorphic chromosome (of the homomorphic pair) to which each allele corresponds. Thus, successful SNP genotyping produces an unordered pair of alleles, where each allele corresponds to one of two haplotypes.

In general, most of the SNPs of a haplotype that correspond to a particular chromosome are sourced from a single chromosome from a parent. However, some of the SNPs from the haplotype may correspond to the parent's other homomorphic chromosome due to chromosomal crossover. Because the genetic information in a particular chromosome of an individual mostly corresponds to a single chromosome of a parent, sequences of SNPs tend to stay relatively intact across generations.

US 2019/0114219 A1 discusses error correction in ancestry classification including obtaining, from a classifier, initial ancestry classifications associated with portions of two phased haplotypes of a chromosome pair of an individual; performing error correction on an initial ancestry classification, including detecting a phasing error in the initial ancestry classifications; and outputting a corrected ancestry classification.

**SUMMARY OF THE INVENTION**

The present invention is defined by the appended independent claims. Preferred embodiments are defined in the dependent claims.

**SUMMARY OF RELATED DISCLOSURE**

The computer implemented system and method described herein assign one or more labels from a set of labels to an input sample genotype dataset. The input sample genotype may be divided into a number of windows, where each window includes a sequence of SNPs from the input sample genotype. Labels may be, for example, ethnicity labels indicating an ancestral origin group. Labels are assigned to the input sample genotype datasets based on an inter-window Hidden Markov Model (HMM) that is built based on the number of windows, each including a sequence of SNPs from the input sample genotype dataset. The inter-window HMM includes a set of states corresponding to each window across the chromosomes of the input sample genotype dataset. Each state may be graphically represented by a node in the HMM.

The inter-window HMM includes a plurality of node groups. Each node group represents a window that corresponds to a segment of genetic data. In each node group, there are a plurality of nodes. Each node in a particular node group represents one of various possible states of the window. The plurality of nodes represent different possible states of the window. Each state includes a first parent label, a second parent label, and a switch label representing a switching of order of the first parent label and the second parent label to account for potential switch errors in the phased haplotypes.

Each node in the inter-window HMM may be associated with an emission probability and each window may be associated with a plurality of emission probabilities. The emission probability represents a likelihood of a particular pair of haplotypes corresponding to the window given the first parent label, the second parent label, and the switch label for each state.

In one embodiment, the emission probability associated with each node may be determined using a convolutional neural network (CNN). The CNN may be trained based on genotype datasets from individuals from a reference panel who are known to belong to an ethnic group. To determine emission probabilities for a target individual, the CNN may use a pair of phased haplotype datasets as input, where the haplotype datasets are obtained by phasing input genotype dataset into a pair of phased haplotype datasets for each window. In other words, the input for a particular CNN corresponding to a window is the phased haplotype datasets for the window and each window may correspond to a different CNN model.

The CNN may include various types of layers, such as convolutional layers, pooling layers, full connected layers, and custom layers. A convolutional layer convolves the input of the layer with one or more kernels to generate abstract feature representations. The kernel may move over the input data and perform operations (e.g. dot product) with the respective sub-region of the input data. As the input for the CNN is a haplotype dataset, which is a sequence of one-dimensional data, the kernel may be a sliding window of a pre-determined length, and the kernel may slide across the input data and perform operations. With determined emission probabilities, an inter-window HMM may be constructed for label assignment.

Each node in the inter-window HMM is also associated with one or more edges, where each edge connects a first node of a first node group to a second node of a second node group. Each edge is associated with a transition probability that represents a likelihood of transition from the first node to the second node.

The inter-window HMM may be computed, built, trained, and updated. For example, transition probabilities of the inter-window HMM may be learned based on expectation-maximization. Using a pair of phased haplotype datasets that are derived from phasing of the input genotype dataset of the individual, a Viterbi path of the inter-window HMM can be determined using a Viterbi algorithm. In other implementations, other types of paths and algorithms may be used instead of Viterbi. The nodes traversed by the Viterbi path each is associated with a first parent label and a second parent label. The composition of the plurality of labels of the nodes can be determined. For example, the distribution of each label in terms of percentage may be determined. The results can be presented as a form of information of the ethnical origins of the individual.

Embodiments according to the invention are in particular disclosed in the attached claims directed to a method and a computer program product, wherein any feature mentioned in one claim category, e.g. method, can be claimed in another claim category, e.g. computer program product, system, storage medium, as well.

BRIEF DESCRIPTION OF THE DRAWINGS

These and other features, aspects, and advantages of the present invention will become better understood with regard to the following description, and accompanying drawings, where:

FIG. 1 illustrates a diagram of a system environment of an example computing system, in accordance with an embodiment.

FIG. 2A is a block diagram of an architecture of an example computing system, in accordance with an embodiment.

FIG. 2B is a block diagram of an ethnicity estimation engine for training and utilizing a model for assigning labels to a genotype, according to one embodiment.

FIG. 2C illustrates differences in reference panels for non-admixed population and admixed population.

FIG. 3A is an example of a haplotype MM, according to an embodiment.

FIG. 3B is an example of a diploid HMM, according to an embodiment.

FIG. 4 is an example of an inter-window HMM, according to an embodiment.

FIG. 5 is an example of a convolutional neural network (CNN) for calculating emission probability for a given window, according to an embodiment.

FIG. 6 is a flowchart illustrating a process for calculating emission probability using CNN.

FIG. 7 is a plot of example label prediction results illustrated with a confusion matrix.

FIG. 8 is a flowchart illustrating a process for calculating emission probability using Markov Models, according to some embodiments.

FIG. 9 is a flowchart illustrating a process for computing transition probabilities, according to some embodiments.

FIG. 10 is a flowchart illustrating a process for assigning labels to a genotype dataset, according to one embodiment.

FIG. 11 is a flowchart illustrating a process for providing information of ethnic origin of an individual based on the individual's genotype dataset, according to one embodiment.

FIG. 12 is a flowchart depicting an example process of generating a synthetic genetic dataset, in accordance with an embodiment.

FIG. 13 is a flowchart depicting an example process of determining ethnic origin composition of an admixed individual, in accordance with an embodiment.

FIG. 14 is the overall process of generating ethnicity ranges and ethnicity confidences, in accordance with an embodiment.

FIG. 15 is a block diagram illustrating an example computer architecture, in accordance with an embodiment.

Note that for purposes of clarity, only one of each item corresponding to a reference numeral is included in most figures, but when implemented multiple instances of any or all of the depicted modules may be employed, as will be appreciated by those of skill in the art.

DETAILED DESCRIPTION

Genetic Data Overview

Individuals may provide deoxyribonucleic acid (DNA) samples (e.g., saliva, skin cells, blood, or other biological matter) for analysis of their genetic data. In one embodiment, an individual uses a sample collection kit to provide a sample from which genetic data can be reliably extracted according to conventional methods. A DNA extraction service can receive the sample and genotype the genetic data, for example by extracting the DNA from the sample and identifying values of SNPs present within the DNA. The result may be referred to as a genotype dataset of the individual. In this disclosure, the result may be an input genotype dataset for further processing based on various processes described in further details below. The genotype dataset is often a diploid genotype. A DNA quality control and matching preparation service may assess data quality of the diploid genotype by checking various attributes such as genotyping call rate, genotyping heterozygosity rate, and agreement between genetic and self-reported gender. The genotype dataset (sometimes also referred to as genotype, or input sample genotype dataset X) is sent (e.g., transmitted through a network) to a ethnicity estimation engine 245. The label determination may receive the genotype from the DNA extraction service or from the DNA quality control and matching preparation service and may store the genotype (e.g., in a database).

A genotype dataset of an individual may include a plurality of SNPs (e.g., say $L$ SNPs). The genotype dataset may be analyzed with focuses on a set of targeted sites of SNPs (e.g., known variable locations of DNA in human genome). Since most SNPs manifest as one of two possible allelic variations within a population (e.g., an SNP may be adenine (A) in some individuals, but cytosine (C) in others), an allele for a particular SNP of a genotype may be referenced by either 0 or 1 (e.g., 0 for A and 1 for C) without loss of generality. Furthermore, although described herein are as using biallelic SNPs (i.e., SNPs that can take on two possible alleles), the methods and systems described herein may be generalized to include multiallelic SNPs (e.g., triallelic SNPs). Additionally, instead of using individual alleles as the basic unit of a genotype dataset, the methods and systems herein may use "mini haplotypes" consisting of multiple alleles as the basic units of data.

A pair of alleles for an SNP in a genotype dataset of an individual may be received without information indicating the homomorphic chromosome to which each allele corresponds. Thus, genotyping data may include in a sequence of $L$ SNPs, each of which contains an unordered pair of values: (0,0) (i.e., homozygous 0), (0,1) (i.e., heterozygous), or (1,1) (i.e., homozygous 1). The first binary value in a pair may be associated with a first parent value and the second binary value may be associated with a second parent value, or vice versa. In some instances, genotyping a particular SNP fails, in which case the alleles for that SNP may be missing. Herein, a genotype dataset may be represented as $G = (G_1, G_2, ..., G_L)$, where each $G_i$ (for $i \in \{1,...,L\}$) is an SNP that has a value of either (0,0), (0,1), (1,1), or missing data.

A genotype dataset $G$ may be divided in $W$ windows, where each window w (for $w \in \{1,...,W\}$) is a sequence of SNPs (i.e., a sub-sequence of $G$). Each window may include a set of sites of SNPs. The sites may correspond to consecutive DNA sequence locations in human chromosome (i.e., every consecutive location of a DNA sequence is a targeted site), but may also be selected sites in which neighboring sites that do not necessarily correspond to neighboring locations in the DNA sequence (e.g., a first SNP site may be at a position A in a DNA sequence while a second SNP site may be at B in the DNA sequence that is hundreds of base pair apart from the position A). In one specific example, each window w includes about 2,000 SNP sites so that the portion of the sequence G corresponding to a window has about 2,000 binary values. The windows may overlap (i.e., share one or more sites of SNPs). For example, a first window may include the first 2,000 sites of SNPs in a chromosome while a second window may include 1,500th to 3,000th sites of SNPs in the chromosome. In one embodiment, a limitation may be imposed such that no window w includes SNPs from more than one chromosome (i.e., from more than one pair of homomorphic chromosomes). For this disclosure, a start point of each window w may be denoted as an SNP index $S_w$ and the length of the window may be denoted as $D_w$. Thus, the sequence of SNPs of the genotype $G$ in window w is $(G_{Sw}, ..., G_{(Sw+Dw-1)})$. Using a phasing algorithm, the genotype G can be phased into a pair of phased haplotype datasets H1 and H2 and the entire sequence can be represented as (H11, H12), (H21, H22), (H31, H32), etc., where Hi1 and Hi2 represent $i$-th SNP.

In some embodiments, genetic composition (e.g., ethnicity composition) of an individual may be determined based on assigning the windows of an input genotype dataset with different labels, such as ethnicity labels. Labels could be any classification labels such as genetic classification labels. In one embodiment, a label corresponds to ancestry from a historical population (e.g., ethnic group). For example, each ethnic group and corresponding label may correspond to a geographic area which the given population historically inhabited. Example areas may be North Africa, Scandinavia, South Asia, etc. For example, a computing system may assign a pair of labels (one being a first parent label such as a patrilineal label and another corresponding to a second parent label such as a matrilineal label) to each window. The labels may be selected from a set of $K$ labels. For example, in the case where the labels are related to ethnic origin, the set of $K$ labels may be African, Asian, European, etc. or be German, Korean, Mexican, etc., depending on the granularity of the classification. A label is an identification of some sequences of haplotypes that are genetically similar. Based on the assigned labels, information of the ethnic origin of the individual may be determined. For example, if 80% of the windows are assigned with a European label, the computing system may provide a statement that the individual is of European origin as an example of information of ethnic origin. The information of ethnic origin may also include statistics of the labels. For example, the computing system may provide a detailed breakdown of the ancestry origins (e.g., 75% European, 20% Asian, and 5% African) of the individual based on the individual's genotype dataset.

The length $D_w$ of each window w may be selected so that each window w likely to corresponds to only a single pair of labels. For example, the length $D_w$ of each window w may be selected so as to have a length of 1-10 centimorgans (cM) or less.

Label Assignment Process Overview

In accordance with an embodiment, a process to characterize a genotype dataset of an individual as a composition of different classifications is conducted through a label assignment process that makes use of different Markov models. A specific example of label assignment is the determination of a composition of ethnicity origins of the individual by assigning different first parent and second parent ethnicity labels to the individual. The genotype dataset is divided into a plurality of segments (which may be called windows). Each window corresponds to a DNA locus that includes a set of SNP sites. Based on the pair of first and second parent ethnicity labels associated with each window, the total compositions of labels of the genotype dataset can be counted. For example, if there are 580 European first parent ethnicity labels assigned to a total of 1000 windows of a genotype data, the genotype data is determined to have about 58% European origin on the first parent side (e.g., on the father side).

The precise assignment of labels to a genotype dataset is conducted by determining a statistically most likely path (commonly referred to as a Viterbi path) of a machine learning model that might be referred to as an inter-window Hidden Markov Model (HMM). In some embodiments, the Viterbi path and a selection (e.g., 1000) of other multiple statistically likely paths (but not as likely as the Viterbi path) that traverse the inter-window HMM are sampled and are used to determine the statistical confidence of the Viterbi path and the final label assignments.

An inter-window HMM includes certain components. First, the inter-window HMM includes hidden states and observations. A hidden state in an HMM may be graphically represented by a node.

In an inter-window HMM in accordance with an embodiment, a hidden state may be a possible condition of the window. Put differently, a window may take one of multiple possible hidden states while different windows may take different hidden states. In the inter-window HMM in accordance with an embodiment, a state is defined by three labels. The first two labels are a first parent label and a second parent label and these two labels are ordered. In other words, for a given inter-window HMM, either the first parent label is consistently first or the second parent label is consistently first among the states. Hence, a first pair of labels of "European, Asian" represents one of the possible states in a window while a second pair of labels of "Asian, European" represent another possible state that is different from the first pair of labels. Each window associated with a particular genotype dataset may take a different state (i.e., different DNA segments of an individual are assigned with different states that have different pairs of ethnicity labels).

The third label that defines a hidden state is a switch label, which represents that, for a particular state, the order of the first parent label and second parent label in the HMM is switched compared to the actual labels in the sample. Put differently, a switching occurs when the HMM assumes a window having a pair of labels in a particular order, but the actual genotype sample at that window has the same pair of labels, but in a reversed order. For example, a label such as "first parent-European, second parent-Asian, switched" means the correct label in the genotype sample is "first parent-Asian, second parent-European." A switch label is used because, in order for first parent label and second parent label to be considered separately, the genotype dataset needs to be phased to generate a pair of haplotype datasets. However, existing phasing methods are often not perfect. The switch label is used to account for the probability that the phasing is incorrect for a particular window.

An observation associated with a hidden state is a possible observable trait, condition, or value in a sample dataset. In an inter-window HMM in accordance with an embodiment, an observation may be the genotype sequence or phased haplotype sequence pair associated with a window. A hidden state is "hidden" because the state is not immediately apparent given the sample dataset. For example, the label "Asian, European, Not-Switched" for a particular window is not immediately apparent given only the sample genotype dataset or the phased pair of haplotype datasets at the particular window. Simply put, when a sequence of SNPs of a sample is ATGCTATAGC... , whether such sequence is inherited from an Asian ancestor, a European ancestor, or another ancestor is not immediately apparent.

Second, the inter-window HMM includes emission probabilities and transition probabilities. A particular hidden state is related to a particular observation by an emission probability. The relationships between different hidden states and different observations might have different values of emission probabilities. A particular hidden state of one window is related to another hidden state of the next window by a transition probability. Graphically, the hidden states in the HMM are represented by nodes that are arranged in node groups (each node group corresponds to a window and the nodes within a node group represent different possible states). An edge that connects two nodes represents a transition with a transition probability.

An emission probability is a probability of an observation being manifested given a particular hidden state. In the inter-window HMM in accordance with an embodiment, an emission probability may represent a likelihood that a particular pair of phased haplotypes is observed in the sample datasets given a particular pair of labels is assigned to the window. Simply put, an emission probability determines what the likelihood is when the sample has the haplotype sequence pairs, for example, "ATGCTATAGC..." and "ATGGTATAGC..." given the window is assigned with, for example, the labels "Asian, European, not-switched." The emission probability represents how likely the DNA in a window comes from an ethnic origin.

An emission probability is associated with each hidden state and is determined based on genotype datasets of reference panels. A reference panel is a collection of individuals' genotype datasets who are known members of an ethnical population. For example, a Germanic reference panel includes genotype datasets of known Germans. At a high level, the determination of an emission probability is based on the input genotype dataset of interest and one or more reference panels to determine the likelihood that the pair of haplotypes presented in each window of the sample genotype dataset comes from the populations of the reference panels. An emission probability may be determined by constructing various models such as Markov Models and Convolutional Neural Networks.

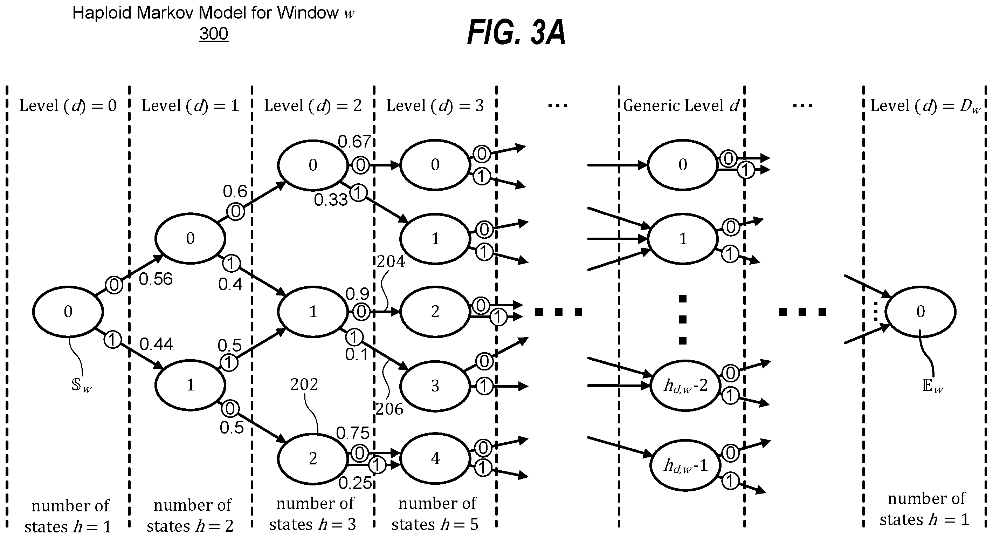

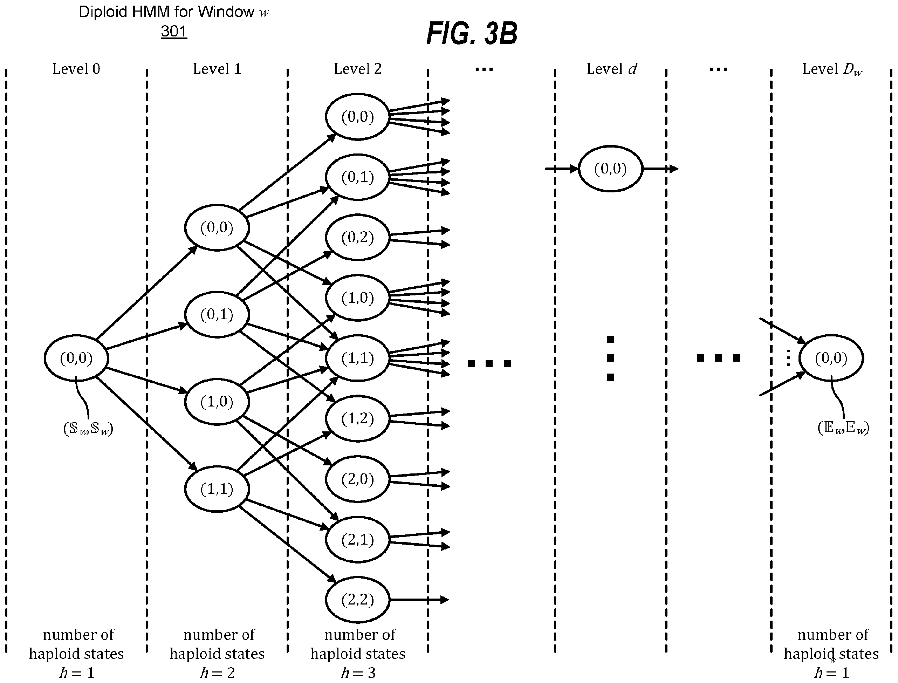

In one embodiment, emission probabilities may be determined by using Markov Models. The determination of an emission probability by using Markov Models involves a series of steps that include creating a haploid Markov Model (MM) and creating a diploid Hidden Markov model (HMM) using the reference panels associated with the labels of the particular hidden state. The details of computing a haploid MM and a diploid HMM for each hidden state are discussed in detail in association with FIGS. 3A and 3B. A series of computations to determine an emission probability may include determinations of one or more intermediate values such as annotations, annotation products, and label pair probabilities.

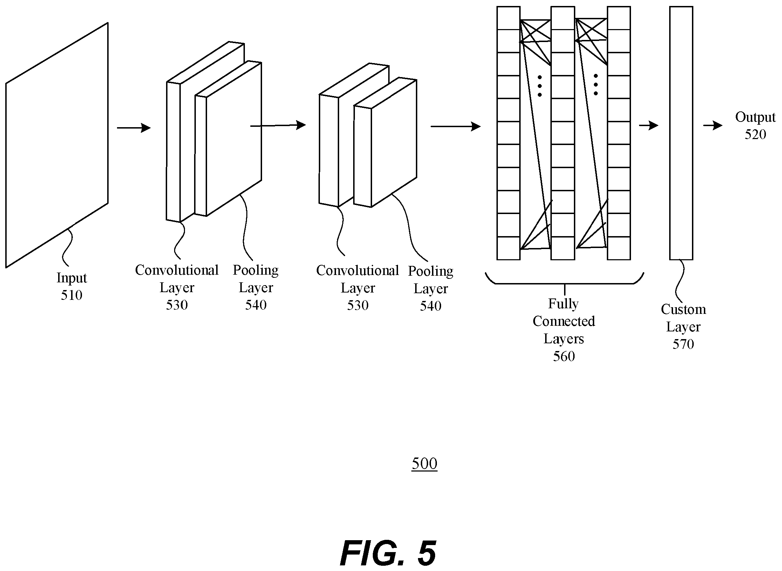

In one embodiment, an emission probability may be determined by training a CNN using genotype datasets of the reference panels. To determine emission probabilities for a target individual, the CNN may use a pair of phased haplotype datasets as input and may generate emission probabilities as output. The CNN may include various types of layers that serve different functionalities. For example, the CNN may include convolutional layers, pooling layers, fully connected layers, and custom layers such as softmax layers, flatten layers and dropout layers. The convolutional layer may have one or more kernels that move across the input dataset and extract abstract feature representation. The convolution results may be passed through subsequent layers and an output may be generated. The CNN may be associated with an objective function that evaluates an error of predictions based on the output from the CNN. Parameters associated with the CNN may be adjusted based on the error. Each iteration may be repeated multiple times until the error is below a desired threshold. Detail of computing an emission probability by training a CNN is further discussed in accordance with FIG. 5.

A transition probability is a probability that a hidden state of a first node group is transitioned to a hidden state of the next node group. In the inter-window HMM in accordance with an embodiment, a transition probability may represent, when a set of labels (e.g., "Asian, European, Not-Switched") is assigned to a window, the likelihood that another set of labels (e.g., same labels "Asian, European, Not-Switched" or different labels "Asian, Asian, Not-Switched") should be assigned to the next window. Humans often inherit a large chunk of DNA from an ancestor. Hence, changes in ethnicity labels are less likely when two windows are next to each other. The ethnicity labels of a window depend on the ethnicity labels of the previous window. The transition probability represents such dependence.

It should be noted that "transition" and "switch" represent different concepts in this disclosure. Transition may refer to a change of one or more of the three labels in a hidden state from one window to the next window. Graphically, in an HMM, a transition is represented by an edge, which is a path going from one node of a node group to another node of the next node group. In contrast, switching is related to a potential incorrect phasing for the haplotypes pair in a particular window. Switching occurs when the HMM assumes that a window is at a state with a pair of labels in a particular order, but the actual genotype sample at that window has the same pair of labels, but in a reversed order. In the HMM, a switch label is one of the label values in a node while a transition is represented by an edge in the HMM.

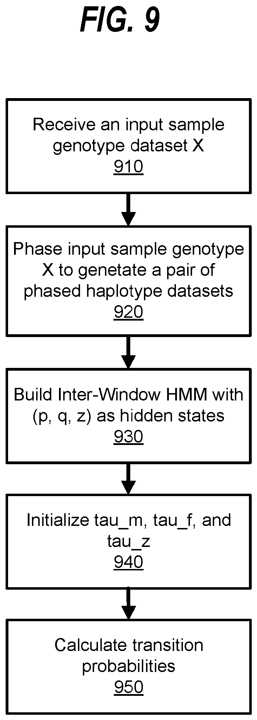

Transition probabilities associated with different edges are determined based on the training of the inter-window HMM until the HMM converges or after a predetermined number of iterations. The training set of the HMM may be sampled from different reference panels such that the training set includes a mix of different ethnicity. In some cases, when a genotype dataset needs to be analyzed, the genotype dataset can first be used to further train the HMM (e.g., as an additional sample of the training set). The determination of transition probabilities is described in further details in FIG. 9.

After the emission probabilities and transition probabilities are determined, the label assignment of a genotype dataset is determined by running a Viterbi algorithm known in the art using the genotype dataset to determine the statistically most likely path of the inter-window HMM (the Viterbi path). The path selects one node for each window (meaning a pair of ethnicity labels are assigned to each window).

The figures (FIGs.) and the following description relate to preferred embodiments by way of illustration only. One of skill in the art may recognize alternative embodiments of the structures and methods disclosed herein as viable alternatives that may be employed without departing from the principles of what is disclosed.

Reference will now be made in detail to several embodiments, examples of which are illustrated in the accompanying figures. It is noted that wherever practicable similar or like reference numbers may be used in the figures and may indicate similar or like functionality. The figures depict embodiments of the disclosed system (or method) for purposes of illustration only. One skilled in the art will readily recognize from the following description that alternative embodiments of the structures and methods illustrated herein may be employed without departing from the principles described herein.

## System Overview

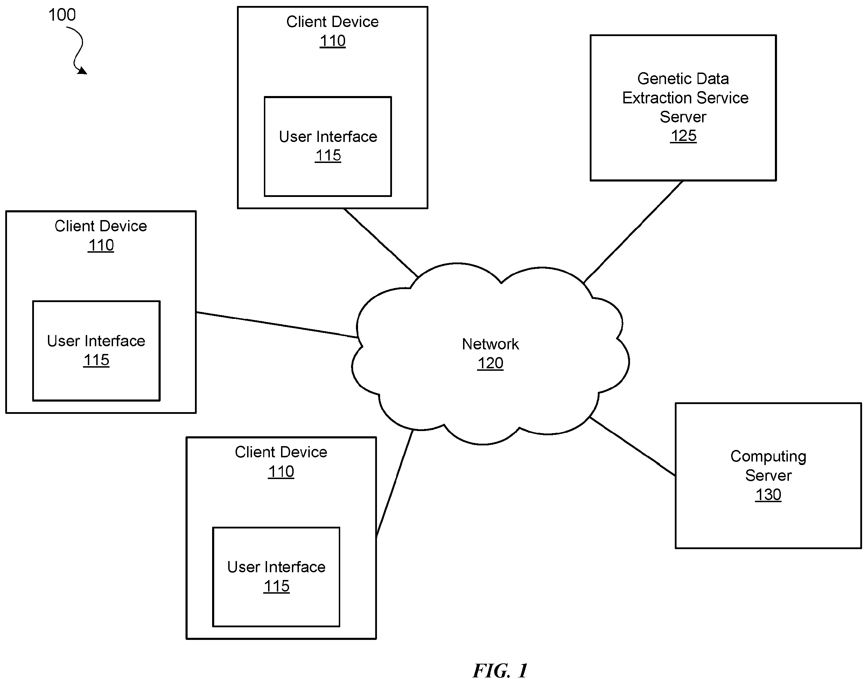

FIG. 1 illustrates a diagram of a system environment 100 of an example computing server 130, in accordance with an embodiment. The system environment 100 shown in FIG. 1 includes one or more client devices 110, a network 120, a genetic data extraction service server 125, and a computing server 130. In various embodiments, the system environment 100 may include fewer or additional components. The system environment 100 may also include different components.

The client devices 110 are one or more computing devices capable of receiving user input as well as transmitting and/or receiving data via a network 120. Example computing devices include desktop computers, laptop computers, personal digital assistants (PDAs), smartphones, tablets, wearable electronic devices (e.g., smartwatches), smart household appliance (e.g., smart televisions, smart speakers, smart home hubs), Internet of Things (IoT) devices or other suitable electronic devices. A client device 110 communicates to other components via the network 120. Users may be customers of the computing server 130 or any individuals who access the system of the computing server 130, such as an online website or a mobile application. In one embodiment, a client device 110 executes an application that launches a graphical user interface (GUI) for a user of the client device 110 to interact with the computing server 130. The GUI may be an example of a user interface 115. A client device 110 may also execute a web browser application to enable interactions between the client device 110 and the computing server 130 via the network 120. In another embodiment, the user interface 115 may take the form of a software application published by the computing server 130 and installed on the user device 110. In yet another embodiment, a client device 110 interacts with the computing server 130 through an application programming interface (API) running on a native operating system of the client device 110, such as IOS or ANDROID.

The network 120 provides connections to the components of the system environment 100 through one or more sub-networks, which may include any combination of local area and/or wide area networks, using both wired and/or wireless communication systems. In one embodiment, a network 120 uses standard communications technologies and/or protocols. For example, a network 120 may include communication links using technologies such as Ethernet, 802.11, worldwide interoperability for microwave access (WiMAX), 3G, 4G, Long Term Evolution (LTE), 5G, code division multiple access (CDMA), digital subscriber line (DSL), etc. Examples of network protocols used for communicating via the network 120 include multiprotocol label switching (MPLS), transmission control protocol/Internet protocol (TCP/IP), hypertext transport protocol (HTTP), simple mail transfer protocol (SMTP), and file transfer protocol (FTP). Data exchanged over a network 120 may be represented using any suitable format, such as hypertext markup language (HTML) or extensible markup language (XML). In some embodiments, all or some of the communication links of a network 120 may be encrypted using any suitable technique or techniques such as secure sockets layer (SSL), transport layer security (TLS), virtual private networks (VPNs), Internet Protocol security (IPsec), etc. The network 120 also includes links and packet switching networks such as the Internet.

Individuals, who may be customers of a company operating the computing server 130, provide biological samples for analysis of their genetic data. Individuals may also be referred to as users. In one embodiment, an individual uses a sample collection kit to provide a biological sample (e.g., saliva, blood, hair, tissue) from which genetic data is extracted and determined according to nucleotide processing techniques such as amplification and sequencing. Amplification may include using polymerase chain reaction (PCR) to amplify segments of nucleotide samples. Sequencing may include sequencing of deoxyribonucleic acid (DNA) sequencing, ribonucleic acid (RNA) sequencing, etc. Suitable sequencing techniques may include Sanger sequencing and massively parallel sequencing such as various next-generation sequencing (NGS) techniques including whole genome sequencing, pyrosequencing, sequencing by synthesis, sequencing by ligation, and ion semiconductor sequencing. In one embodiment, a set of SNPs (e.g., 300,000) that are shared between different array platforms (e.g., Illumina OmniExpress Platform and Illumina HumanHap 650Y Platform) may be obtained as the genetic data. Genetic data extraction service server 125 receives biological samples from users of the computing server 130. The genetic data extraction service server 125 performs sequencing of the biological samples and determines the base pair sequences of the individuals. The genetic data extraction service server 125 generates the genetic data of the individuals based on the sequencing results. The genetic data may include data sequenced from DNA or RNA and may include base pairs from coding and/or noncoding regions of DNA.

The genetic data may take different forms and include information regarding various biomarkers of an individual. For example, in one embodiment, the genetic data may be the base pair sequence of an individual. The base pair sequence may include the whole genome or a part of the genome such as certain genetic loci of interest. In another embodiment, the genetic data extraction service server 125 may determine genotypes from sequencing results, for example by identifying genotype values of single nucleotide polymorphisms (SNPs) present within the DNA. The results in this example may include a sequence of genotypes corresponding to various SNP sites. A SNP site may also be referred to as a SNP loci. A genetic locus is a segment of a genetic sequence. A locus can be a single site or a longer stretch. The segment can be a single base long or multiple bases long. In one embodiment, the genetic data extraction service server 125 may perform data pre-processing of the genetic data to convert raw sequences of base pairs to sequences of genotypes at target SNP sites. Since a typical human genome may differ from a reference human genome at only several million SNP sites (as opposed to billions of base pairs in the whole genome), the genetic data extraction service server 125 may extract only the genotypes at a set of target SNP sites and transmit the extracted data to the computing server 130 as the genetic dataset of an individual. SNPs, base pair sequence, genotype, haplotype, RNA sequences, protein sequences, phenotypes are examples of biomarkers.

The computing server 130 performs various analyses of the genetic data, genealogical data, and users' survey responses to generate results regarding the phenotypes and genealogy of users of computing server 130. Depending on the embodiments, the computing server 130 may also be referring to as an online server, a personal genetic service server, a genealogy server, a family tree building server, and/or a social networking system. The computing server 130 receives genetic data from the genetic data extraction service server 125 and stores the genetic data in the data store of the computing server 130. The computing server 130 may analyze the data to generate results regarding the genetics or genealogy of users. The results regarding the genetics or genealogy of users may include the ethnicity compositions of users, paternal and maternal genetic analysis, identification or suggestion of potential family relatives, ancestor information, analyses of DNA data, potential or identified traits such as phenotypes of users (e.g., diseases, appearance traits, other genetic characteristics, and other non-genetic characteristics including social characteristics), etc. The computing server 130 may present or cause the user interface 115 to present the results to the users through a GUI displayed at the client device 110. The results may include graphical elements, textual information, data, charts, and other elements such as family trees.

In one embodiment, the computing server 130 also allows various users to create one or more genealogical profiles of the user. The genealogical profile may include a list of individuals (e.g., ancestors, relatives, friends, and other people of interest) who are added or selected by the user or suggested by the computing server 130 based on the genealogical records and/or genetic records. The user interface 115 controlled by or in communication with the computing server 130 may display the individuals in a list or as a family tree such as in the form of a pedigree chart. In one embodiment, subject to user's privacy setting and authorization, the computing server 130 may allow information generated from the user's genetic dataset to be linked to the user profile and to one or more of the family trees. The users may also authorize the computing server 130 to analyze their genetic dataset and allow their profiles to be discovered by other users.

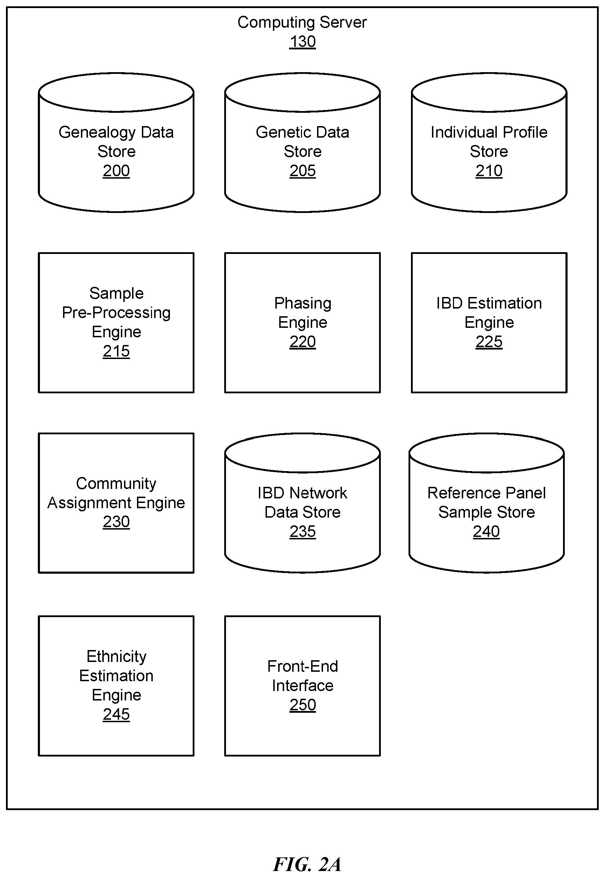

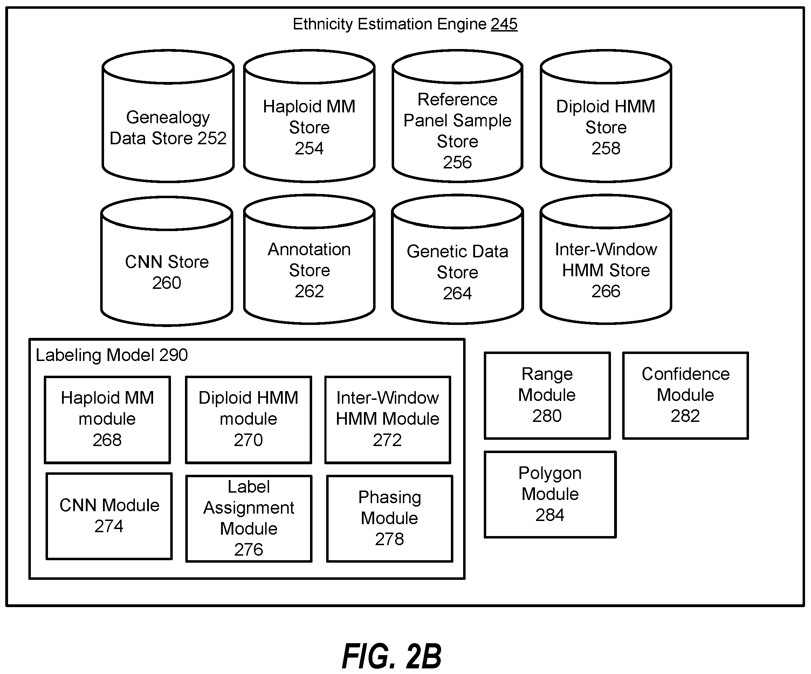

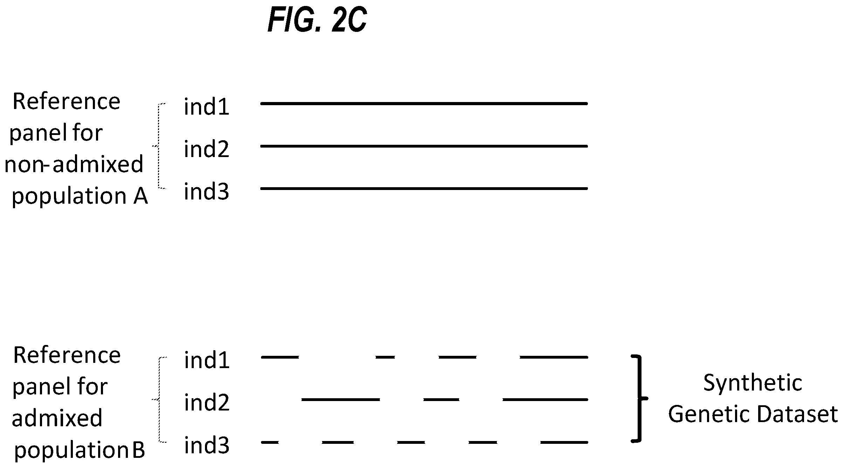

FIG. 2A is a block diagram of an architecture of an example computing server 130, in accordance with an embodiment. In the embodiment shown in FIG. 2, the computing server 130 includes a genealogy data store 200, a genetic data store 205, an individual profile store 210, a sample pre-processing engine 215, a phasing engine 220, an identity by descent (IBD) estimation engine 225, a community assignment engine 230, an IBD network data store 235, a reference panel sample store 240, an ethnicity estimation engine 245, and a front-end interface 250. The functions of the computing server 130 may be distributed among the elements in a different manner than described. In various embodiments, the computing server 130 may include different components and fewer or additional components. Each of the various data stores may be a single storage device, a server controlling multiple storage devices, or a distributed network that is accessible through multiple nodes (e.g., a cloud storage system).

The computing server 130 stores various data of different individuals, including genetic data, genealogical data, and survey response data. The computing server 130 processes the genetic data of users to identify shared identity-by-descent (IBD) segments between individuals. The genealogical data and survey response data may be part of user profile data. The amount and type of user profile data stored for each user may vary based on the information of a user, which is provided by the user as she creates an account and profile at a system operated by the computing server 130 and continues to build her profile, family tree, and social network at the system and to link her profile with her genetic data. Users may provide data via the user interface 115 of a client device 110. Initially and as a user continues to build her genealogical profile, the user may be prompted to answer questions related to basic information of the user (e.g., name, date of birth, birthplace, etc.) and later on more advanced questions that may be useful for obtaining additional genealogical data. The computing server 130 may also include survey questions regarding various traits of the users such as the users' phenotypes, characteristics, preferences, habits, lifestyle, environment, etc.

Genealogical data may be stored in the genealogical data store 200 and may include various types of data that are related to tracing family relatives of users. Examples of genealogical data include names (first, last, middle, suffixes), gender, birth locations, date of birth, date of death, marriage information, spouse's information kinships, family history, dates and places for life events (e.g., birth and death), other vital data, and the like. In some instances, family history can take the form of a pedigree of an individual (e.g., the recorded relationships in the family). The family tree information associated with an individual may include one or more specified nodes. Each node in the family tree represents the individual, an ancestor of the individual who might have passed down genetic material to the individual, and the individual's other relatives including siblings, cousins, offspring in some cases. Genealogical data may also include connections and relationships among users of the computing server 130. The information related to the connections among a user and her relatives that may be associated with a family tree may also be referred to as pedigree data or family tree data.

In addition to user-input data, genealogical data may also take other forms that are obtained from various sources such as public records and third-party data collectors. For example, genealogical records from public sources include birth records, marriage records, death records, census records, court records, probate records, adoption records, obituary records, etc. Likewise, genealogical data may include data from one or more of a pedigree of an individual, the Ancestry World Tree system, a Social Security Death Index database, the World Family Tree system, a birth certificate database, a death certificate database, a marriage certificate database, an adoption database, a draft registration database, a veterans database, a military database, a property records database, a census database, a voter registration database, a phone database, an address database, a newspaper database, an immigration database, a family history records database, a local history records database, a business registration database, a motor vehicle database, and the like.

Furthermore, the genealogical data store 200 may also include relationship information inferred from the genetic data stored in the genetic data store 205 and information received from the individuals. For example, the relationship information may indicate which individuals are genetically related, how they are related, how many generations back they share common ancestors, lengths and locations of IBD segments shared, which genetic communities an individual is a part of, variants carried by the individual, and the like.

The computing server 130 maintains genetic datasets of individuals in the genetic data store 205. A genetic dataset of an individual may be a digital dataset of nucleotide data (e.g., SNP data) and corresponding metadata. A genetic dataset may contain data of the whole or portions of an individual's genome. The genetic data store 205 may store a pointer to a location associated with the genealogical data store 200 associated with the individual. A genetic dataset may take different forms. In one embodiment, a genetic dataset may take the form of a base pair sequence of the sequencing result of an individual. A base pair sequence dataset may include the whole genome of the individual (e.g., obtained from a whole-genome sequencing) or some parts of the genome (e.g., genetic loci of interest).

In another embodiment, a genetic dataset may take the form of sequences of genetic markers. Examples of genetic markers may include target SNP loci (e.g., allele sites) filtered from the sequencing results. A SNP locus that is single base pair long may also be referred to a SNP site. A SNP locus may be associated with a unique identifier. The genetic dataset may be in a form of a diploid data that includes a sequencing of genotypes, such as genotypes at the target SNP loci, or the whole base pair sequence that includes genotypes at known SNP loci and other base pair sites that are not commonly associated with known SNPs. The diploid dataset may be referred to as a genotype dataset or a genotype sequence. Genotype may have a different meaning in various contexts. In one context, an individual's genotype may refer to a collection of diploid alleles of an individual. In other contexts, a genotype may be a pair of alleles present on two chromosomes for an individual at a given genetic marker such as a SNP site.

A genotype at a SNP site may include a pair of alleles. The pair of alleles may be homozygous (e.g., A-A or G-G) or heterozygous (e.g., A-T, C-T). Instead of storing the actual nucleotides, the genetic data store 205 may store genetic data that are converted to bits. For a given SNP site, oftentimes only two nucleotide alleles (instead of all 4) are observed. As such, a 2-bit number may represent a SNP site. For example, 00 may represent homozygous first alleles, 11 may represent homozygous second alleles, and 01 or 10 may represent heterozygous alleles. A separate library may store what nucleotide corresponds to the first allele and what nucleotide corresponds to the second allele at a given SNP site.

A diploid dataset may also be phased into two sets of haploid data, one corresponding to a first parent side and another corresponding to a second parent side. The phased datasets may be referred to as haplotype datasets or haplotype sequences. Similar to genotype, haplotype may have a different meaning in various contexts. In one context, a haplotype may also refer to a collection of alleles that corresponds to a genetic segment. In other contexts, a haplotype may refer to a specific allele at a SNP site. For example, a sequence of haplotypes may refer to a sequence of alleles of an individual that are inherited from a parent.

The individual profile store 210 stores profiles and related metadata associated with various individuals appeared in the computing server 130. A computing server 130 may use unique individual identifiers to identify various users and other non-users that might appear in other data sources such as ancestors or historical persons who appear in any family tree or genealogical database. A unique individual identifier may a hash of certain identification information of an individual, such as a user's account name, user's name, date of birth, location of birth, or any suitable combination of the information. The profile data related to an individual may be stored as metadata associated with an individual's profile. For example, the unique individual identifier and the metadata may be stored as a key-value pair using the unique individual identifier as a key.

An individual's profile data may include various kinds of information related to the individual. The metadata about the individual may include one or more pointer associating genetic datasets such as genotype and phased haplotype data of the individual that are saved in the genetic data store 205. The metadata about the individual may also individual information related to family trees and pedigree datasets that include the individual. The profile data may further include declarative information about the user that was authorized by the user to be shared and may also include information inferred by the computing server 130. Other examples of information stored in a user profile may include biographic, demographic, and other types of descriptive information such as work experience, educational history, gender, hobbies, or preferences, location and the like. In one embodiment, the user profile data may also include one or more photos of the users and photos of relatives (e.g., ancestors) of the users that are uploaded by the users. A user may authorize the computing server 130 to analyze one or more photos to extract information, such as user's or relative's appearance traits (e.g., blue eyes, curved hair, etc.), from the photos. The appearance traits and other information extracted from the photos may also be saved in the profile store. In some cases, the computing server may allow users to upload many different photos of the users, their relatives, and even friends. User profile data may also be obtained from other suitable sources, including historical records (e.g., records related to an ancestor), medical records, military records, photographs, other records indicating one or more traits, and other suitable recorded data.

For example, the computing server 130 may present various survey questions to its users from time to time. The responses to the survey questions may be stored at individual profile store 210. The survey questions may be related to various aspects of the users and the users' families. Some survey questions may be related to users' phenotypes, while other questions may be related to environmental factors of the users.

Survey questions may concern health or disease-related phenotypes, such as questions related to the presence or absence of genetic diseases or disorders, inheritable diseases or disorders, or other common diseases or disorders that have family history as one of the risk factors, questions regarding any diagnosis of increased risk of any diseases or disorders, and questions concerning wellness-related issues such as family history of obesity, family history of causes of death, etc. The diseases identified by the survey questions may be related to single-gene diseases or disorders that are caused by a single-nucleotide variant, an insertion, or a deletion. The diseases identified by the survey questions may also be multifactorial inheritance disorders that may be caused by a combination of environmental factors and genes. Examples of multifactorial inheritance disorders may include heart disease, Alzheimer's diseases, diabetes, cancer, and obesity. The computing server 130 may obtain data of a user's disease-related phenotypes from survey questions of health history of the user and her family and also from health records uploaded by the user.

Survey questions also may be related to other types of phenotypes such as appearance traits of the users. A survey regarding appearance traits and characteristics may include questions related to eye color, iris pattern, freckles, chin types, finger length, dimple chin, earlobe types, hair color, hair curl, skin pigmentation, susceptibility to skin burn, bitter taste, male baldness, baldness pattern, presence of unibrow, presence of wisdom teeth, height, and weight. A survey regarding other traits also may include questions related to users' taste and smell such as the ability to taste bitterness, asparagus smell, cilantro aversion, etc. A survey regarding traits may further include questions related to users' body conditions such as lactose tolerance, caffeine consumption, malaria resistance, norovirus resistance, muscle performance, alcohol flush, etc. Other survey questions regarding a person's physiological or psychological traits may include vitamin traits and sensory traits such as ability to sense an asparagus metabolite. Traits may also be collected from historical records, electronic health records and electronic medical records.

The computing server 130 also may present various survey questions related to environmental factors of users. In this context, an environmental factor may be a factor that is not directly connected to the genetics of the users. Environmental factors may include users' preferences, habits, and lifestyle. For example, a survey regarding users' preferences may include questions related to things and activities that users like or dislike, such as types of music a user enjoys, dancing preference, party-going preference, certain sports that a user plays, video games preferences, etc. Other questions may be related to the users' diet preference such as like or dislike a certain type of food (e.g., ice cream, egg). A survey related to habits and lifestyle may include questions regarding smoking habits, alcohol consumption and frequency, daily exercise duration, sleeping habits (e.g., morning person versus night person), sleeping cycles and problems, hobbies, and travel preferences. Additional environmental factors may include diet amount (calories, macronutrients), physical fitness abilities (e.g. stretching, flexibility, heart rate recovery), family type (adopted family or not, has siblings or not, lived with extended family during childhood), property and item ownership (has home or rents, has smartphone or doesn't, has car or doesn't).

Surveys also may be related to other environmental factors such as geographical, social-economic, or cultural factors. Geographical questions may include questions related to the birth location, family migration history, town, or city of users' current or past residence. Social-economic questions may be related to users' education level, income, occupations, self-identified demographic groups, etc. Questions related to culture may concern users' native language, language spoken at home, customs, dietary practices, etc. Other questions related to users' cultural and behavioral questions are also possible.

For any survey questions asked, the computing server 130 may also ask an individual the same or similar questions regarding the traits and environmental factors of the ancestors, family members, other relatives or friends of the individual. For example, a user may be asked about the native language of the user and the native languages of the user's parents and grandparents. A user may also be asked about the health history of his or her family members.

In addition to storing the survey data in the individual profile store 210, the computing server 130 may store some responses that correspond to data related to genealogical and genetics respectively to genealogical data store 200 and genetic data store 205.

The user profile data, photos of users, survey response data, the genetic data, and the genealogical data may subject to the privacy and authorization setting from the users to specify any data related to the users can be accessed, stored, obtained, or otherwise used. For example, when presented with a survey question, a user may select to answer or skip the question. The computing server 130 may present users from time to time information regarding users' selection of the extent of information and data shared. The computing server 130 also may maintain and enforce one or more privacy settings for users in connection with the access of the user profile data, photos, genetic data, and other sensitive data. For example, the user may pre-authorize the access of the data and may change the setting as wish. The privacy settings also may allow a user to specify (e.g., by opting out, by not opting in) whether the computing server 130 may receive, collect, log, or store particular data associated with the user for any purpose. A user may restrict her data at various levels. For example, in one level, the data may not be accessed by the computing server 130 for purposes other than displaying the data in the user's own profile. On another level, the user may authorize anonymization of her data and participate in studies and researches conducted by the computing server 130 such as a large scale genetic study. In yet another level, the user may turn some portions of her genealogical data public to allow the user to be discovered by other users (e.g., potential relatives) and be connected in one or more family trees. Access or sharing of any information or data in the computing server 130 may also be subject to one or more similar privacy policies. A user's data and content objects in the computing server 130 may also be associated with different levels of restriction. The computing server 130 may also provide various notification feature to inform and remind users of their privacy and access settings. For example, when privacy settings for a data entry allow a particular user or other entities to access the data, the data may be described as being "visible," "public," or other suitable labels, in contrary to a "private" label.

In some cases, the computing server 130 may have a heightened privacy protection on certain types of data and data related to certain vulnerable groups. In some cases, the heightened privacy settings may strictly prohibit the use, analysis, sharing of data related to a certain vulnerable group. In other cases, the heightened privacy settings may specify that data subject to those settings require prior approval for access, publication, or other use. In some cases, the computing server 130 may provide the heightened privacy as a default setting for certain types of data, such as genetic data or any data that the user marks as sensitive. The user may opt in for sharing of those data or change the default privacy settings. In other cases, the heightened privacy settings may apply across the board for all data of certain groups of users. For example, if the computing server 130 determines that the user is a minor or has recognized that a picture of a minor is uploaded, the computing server 130 may designate all profile data associated with the minor as sensitive. In those cases, the computing server 130 may have one or more extra steps in seeking and confirming any sharing or use of the sensitive data.

The sample pre-processing engine 215 receives and pre-processes data received from various sources to change the data into a format used by the computing server 130. For genealogical data, the sample pre-processing engine 215 may receive data from an individual via the user interface 115 of the client device 110. To collect the user data (e.g., genealogical and survey data), the computing server 130 may cause an interactive user interface on the client device 110 to display interface elements in which users can provide genealogical data and survey data. Additional data may be obtained from scans of public records. The data may be manually provided or automatically extracted via, for example, optical character recognition (OCR) performed on census records, town or government records, or any other item of printed or online material. Some records may be obtained by digitalizing written records such as older census records, birth certificates, death certificates, etc.

The sample pre-processing engine 215 may also receive raw data from genetic data extraction service server 125. The genetic data extraction service server 125 may perform laboratory analysis of biological samples of users and generate sequencing results in the form of digital data. The sample pre-processing engine 215 may receive the raw genetic datasets from the genetic data extraction service server 125. The human genome mutation rate is estimated to be $1.1 * 10^{-8}$ per site per generation. This leads to a variant approximately every 300 base pairs. Most of the mutations that are passed down to descendants are related to single-nucleotide polymorphism (SNP). SNP is a substitution of a single nucleotide that occurs at a specific position in the genome. The sample pre-processing engine 215 may convert the raw base pair sequence into a sequence of genotypes of target SNP sites. Alternatively, the pre-processing of this conversion may be performed by the genetic data extraction service server 125. The sample pre-processing engine 215 identifies autosomal SNPs in an individual's genetic dataset. In one embodiment, the SNPs may be autosomal SNPs. In one embodiment, 700,000 SNPs may be identified in an individual's data and may be stored in genetic data store 205.

Alternatively, in one embodiment, a genetic dataset may include at least 10,000 SNP sites. In another embodiment, a genetic dataset may include at least 100,000 SNP sites. In yet another embodiment, a genetic dataset may include at least 300,000 SNP sites. In yet another embodiment, a genetic dataset may include at least 1,000,000 SNP sites. The sample pre-processing engine 215 may also convert the nucleotides into bits. The identified SNPs, in bits or in other suitable formats, may be provided to the phasing engine 220 which phases the individual's diploid genotypes to generate a pair of haplotypes for each user.

The phasing engine 220 phases diploid genetic dataset into a pair of haploid genetic datasets and may perform imputation of SNP values at certain sites whose alleles are missing. An individual's haplotype may refer to a collection of alleles (e.g., a sequence of alleles) that are inherited from a parent.

Phasing may include a process of determining the assignment of alleles (particularly heterozygous alleles) to chromosomes. Owing to sequencing conditions and other constraints, a sequencing result often includes data regarding a pair of alleles at a given SNP locus of a pair of chromosomes but may not be able to distinguish which allele belongs to which specific chromosome. The phasing engine 220 uses a genotype phasing algorithm to assign one allele to a first chromosome and another allele to another chromosome. The genotype phasing algorithm may be developed based on an assumption of linkage disequilibrium (LD), which states that haplotype in the form of a sequence of alleles tends to cluster together. The phasing engine 220 is configured to generate phased sequences that are also commonly observed in many other samples. Put differently, haplotype sequences of different individuals tend to cluster together. A haplotype-cluster model may be generated to determine the probability distribution of a haplotype that includes a sequence of alleles. The haplotype-cluster model may be trained based on labeled data that includes known phased haplotypes from a trio (parents and a child). A trio is used as a training sample because the correct phasing of the child is almost certain by comparing the child's genotypes to the parent's genetic datasets. The haplotype-cluster model may be generated iteratively along with the phasing process with a large number of unphased genotype datasets. The haplotype-cluster model may also be used to impute one or more missing data.

By way of example, the phasing engine 220 may use a directed acyclic graph model such as a hidden Markov model (HMM) to perform phasing of a target genotype dataset. The directed acyclic graph may include multiple levels, each level having multiple nodes representing different possibilities of haplotype clusters. An emission probability of a node, which may represent the probability of having a particular haplotype cluster given an observation of the genotypes may be determined based on the probability distribution of the haplotype-cluster model. A transition probability from one node to another may be initially assigned to a non-zero value and be adjusted as the directed acyclic graph model and the haplotype-cluster model are trained. Various paths are possible in traversing different levels of the directed acyclic graph model. The phasing engine 220 determines a statistically likely path, such as the most probable path or a probable path that is at least more likely than 95% of other possible paths, based on the transition probabilities and the emission probabilities. A suitable dynamic programming algorithm such as the Viterbi algorithm may be used to determine the path. The determined path may represent the phasing result. U.S. Patent Application No. 15/519,099, entitled "Haplotype Phasing Models," filed on October 19, 2015, describes one possible embodiment of haplotype phasing.

The IBD estimation engine 225 estimates the amount of shared genetic segments between a pair of individuals based on phased genotype data (e.g., haplotype datasets) that are stored in the genetic data store 205. IBD segments may be segments identified in a pair of individuals that are putatively determined to be inherited from a common ancestor. The IBD estimation engine 225 retrieves a pair of haplotype datasets for each individual. The IBD estimation engine 225 may divide each haplotype dataset sequence into a plurality of windows. Each window may include a fixed number of SNP sites (e.g., about 100 SNP sites). The IBD estimation engine 225 identifies one or more seed windows in which the alleles at all SNP sites in at least one of the phased haplotypes between two individuals are identical. The IBD estimation engine 225 may expand the match from the seed windows to nearby windows until the matched windows reach the end of a chromosome or until a homozygous mismatch is found, which indicates the mismatch is not attributable to potential errors in phasing or in imputation. The IBD estimation engine 225 determines the total length of matched segments, which may also be referred to as IBD segments. The length may be measured in the genetic distance in the unit of centimorgans (cM). A unit of centimorgan may be a genetic length. For example, two genomic positions that are one cM apart may have a 1% chance during each meiosis of experiencing a recombination event between the two positions. The computing server 130 may save data regarding individual pairs who share a length of IBD segments exceeding a predetermined threshold (e.g., 6 cM), in a suitable data store such as in the genealogical data store 200. U.S. Patent Application No. 14/029,765, entitled "Identifying Ancestral Relationships Using a Continuous stream of Input," filed on September 17, 2013, and U.S. Patent Application No. 15/519,104, entitled "Reducing Error in Predicted Genetic Relationships," filed on April 13, 2017, describe example embodiments of IBD estimation.

Typically, individuals who are closely related share a relatively large number of IBD segments, and the IBD segments tend to have longer lengths (individually or in aggregate across one or more chromosomes). In contrast, individuals who are more distantly related share relatively fewer IBD segments, and these segments tend to be shorter (individually or in aggregate across one or more chromosomes). For example, while close family members often share upwards of 71 cM of IBD (e.g., third cousins), more distantly related individuals may share less than 12 cM of IBD. The extent of relatedness in terms of IBD segments between two individuals may be referred to as IBD affinity. For example, the IBD affinity may be measured in terms of the length of IBD segments shared between two individuals.

Community assignment engine 230 assigns individuals to one or more genetic communities based on the genetic data of the individuals. A genetic community may correspond to an ethnic origin or a group of people descended from a common ancestor. The granularity of genetic community classification may vary depending on embodiments and methods used in assigning communities. For example, in one embodiment, the communities may be African, Asian, European, etc. In another embodiment, the European community may be divided into Irish, German, Swedes, etc. In yet another embodiment, the Irish may be further divided into Irish in Ireland, Irish immigrated to America in 1800, Irish immigrated to America in 1900, etc. The community classification may also depend on whether a population is admixed or unadmixed. For an admixed population, the classification may further be divided based on different ethnic origins in a geographical region.

Community assignment engine 230 may assign individuals to one or more genetic communities based on their genetic datasets using machine learning models trained by unsupervised learning or supervised learning. In an unsupervised approach, the community assignment engine 230 may generate data representing a partially connected undirected graph. In this approach, the community assignment engine 230 represents individuals as nodes. Some nodes are connected by edges whose weights are based on IBD affinity between two individuals represented by the nodes. For example, if the total length of two individuals' shared IBD segments does not exceed a predetermined threshold, the nodes are not connected. The edges connecting two nodes are associated with weights that are measured based on the IBD affinities. The undirected graph may be referred to as an IBD network. The community assignment engine 230 uses clustering techniques such as modularity measurement (e.g., the Louvain method) to classify nodes into different clusters in the IBD network. Each cluster may represent a community. The community assignment engine 230 may also determine sub-clusters, which represent sub-communities. The computing server 130 saves the data representing the IBD network and clusters in the IBD network data store 235. U.S. Patent Application No. 15/168,011, entitled "Discovering Population Structure from Patterns of Identity-By-Descent," filed on May 28, 2016, describes one possible embodiment of community detection and assignment.

The community assignment engine 230 may also assign communities using supervised techniques. For example, genetic datasets of known genetic communities (e.g., individuals with confirmed ethnic origins) may be used as training sets that have labels of the genetic communities. Supervised machine learning classifiers, such as logistic regressions, support vector machines, random forest classifiers, and neural networks may be trained using the training set with labels. A trained classifier may distinguish binary or multiple classes. For example, a binary classifier may be trained for each community of interest to determine whether a target individual's genetic dataset belongs or does not belong to the community of interest. A multi-class classifier such as a neural network may also be trained to determine whether the target individual's genetic dataset most likely belongs to one of several possible genetic communities.

Reference panel sample store 240 stores reference panel samples for different genetic communities. A reference panel sample is a genetic data of an individual whose genetic data is the most representative of a genetic community. The genetic data of individuals with the typical alleles of a genetic community may serve as reference panel samples. For example, some alleles of genes may be over-represented (e.g., being highly common) in a genetic community. Some genetic datasets include alleles that are commonly present among members of the community. Reference panel samples may be used to train various machine learning models in classifying whether a target genetic dataset belongs to a community, in determining the ethnic composition of an individual, and in determining the accuracy in any genetic data analysis, such as by computing a posterior probability of a classification result from a classifier.

A reference panel sample may be identified in different ways. In one embodiment, an unsupervised approach in community detection may apply the clustering algorithm recursively for each identified cluster until the sub-clusters contain a number of nodes that is smaller than a threshold (e.g., contains fewer than 1000 nodes). For example, the community assignment engine 230 may construct a full IBD network that includes a set of individuals represented by nodes and generate communities using clustering techniques. The community assignment engine 230 may randomly sample a subset of nodes to generate a sampled IBD network. The community assignment engine 230 may recursively apply clustering techniques to generate communities in the sampled IBD network. The sampling and clustering may be repeated for different randomly generated sampled IBD networks for various runs. Nodes that are consistently assigned to the same genetic community when sampled in various runs may be classified as a reference panel sample. The community assignment engine 230 may measure the consistency in terms of a predetermined threshold. For example, if a node is classified to the same community 95% (or another suitable threshold) of times whenever the node is sampled, the genetic dataset corresponding to the individual represented by the node may be regarded as a reference panel sample. Additionally, or alternatively, the community assignment engine 230 may select N most consistently assigned nodes as a reference panel for the community.

Other ways to generate reference panel samples are also possible. For example, the computing server 130 may collect a set of samples and gradually filter and refine the samples until high-quality reference panel samples are selected. For example, a candidate reference panel sample may be selected from an individual whose recent ancestors are born at a certain birthplace. The computing server 130 may also draw sequence data from the Human Genome Diversity Project (HGDP). Various candidates may be manually screened based on their family trees, relatives' birth location, other quality control. Principal component analysis may be used to creates clusters of genetic data of the candidates. Each cluster may represent an ethnicity. The predictions of the ethnicity of those candidates may be compared to the ethnicity information provided by the candidates to perform further screening.

The ethnicity estimation engine 245 estimates the ethnicity composition of a genetic dataset of a target individual. The genetic datasets used by the ethnicity estimation engine 245 may be genotype datasets or haplotype datasets. For example, the ethnicity estimation engine 245 estimates the ancestral origins (e.g., ethnicity) based on the individual's genotypes or haplotypes at the SNP sites. To take a simple example of three ancestral populations corresponding to African, European and Native American, an admixed user may have nonzero estimated ethnicity proportions for all three ancestral populations, with an estimate such as [0.05, 0.65, 0.30], indicating that the user's genome is 5% attributable to African ancestry, 65% attributable to European ancestry and 30% attributable to Native American ancestry. The ethnicity estimation engine 245 generates the ethnic composition estimate and stores the estimated ethnicities in a data store of computing server 130 with a pointer in association with a particular user.

In one embodiment, the ethnicity estimation engine 245 divides a target genetic dataset into a plurality of windows (e.g., about 1000 windows). Each window includes a small number of SNPs (e.g., 300 SNPs). The ethnicity estimation engine 245 may use a directed acyclic graph model to determine the ethnic composition of the target genetic dataset. The directed acyclic graph may represent a trellis of an inter-window hidden Markov model (HMM). The graph includes a sequence of a plurality of node group. Each node group, representing a window, includes a plurality of nodes. The nodes representing different possibilities of labels of genetic communities (e.g., ethnicities) for the window. A node may be labeled with one or more ethnic labels. For example, a level includes a first node with a first label representing the likelihood that the window of SNP sites belongs to a first ethnicity and a second node with a second label representing the likelihood that the window of SNPs belongs to a second ethnicity. Each level includes multiple nodes so that there are many possible paths to traverses the directed acyclic graph.

The nodes and edges in the directed acyclic graph may be associated with different emission probabilities and transition probabilities. An emission probability associated with a node represents the likelihood that the window belongs to the ethnicity labeling the node given the observation of SNPs in the window. The ethnicity estimation engine 245 may determine the emission probabilities by comparing SNPs in the window corresponding to the target genetic dataset to corresponding SNPs in the windows in various reference panel samples of different genetic communities stored in the reference panel sample store 240. The ethnicity estimation engine 245 may determine the emission probabilities via various models including Markov Models and machine learning models such as a CNN. The transition probability between two nodes represents the likelihood of transition from one node to another across two levels. The ethnicity estimation engine 245 determines a statistically likely path, such as the most probable path or a probable path that is at least more likely than 95% of other possible paths, based on the transition probabilities and the emission probabilities. A suitable dynamic programming algorithm such as the Viterbi algorithm or the forward-backward algorithm may be used to determine the path. After the path is determined, the ethnicity estimation engine 245 determines the ethnic composition of the target genetic dataset by determining the label compositions of the nodes that are included in the determined path. U.S. Patent Application No. 15/209,458, entitled "Local Genetic Ethnicity Determination System," filed on July 13, 2016, describes an example embodiment of ethnicity estimation.

The front-end interface 250 may display various results determined by the computing server 130. The results and data may include the IBD affinity between a user and another individual, the community assignment of the user, the ethnicity estimation of the user, phenotype prediction and evaluation, genealogical data search, family tree and pedigree, relative profile and other information. The front-end interface 250 may be a graphical user interface (GUI) that displays various information and graphical elements. The front-end interface 250 may take different forms. In one case, the front-end interface 250 may be a software application that can be displayed at an electronic device such as a computer or a smartphone. The software application may be developed by the entity controlling the computing server 130 and be downloaded and installed at the client device 110. In another case, the front-end interface 250 may take the form of a webpage interface of the computing server 130 that allows users to access their family tree and genetic analysis results through web browsers. In yet another case, the front-end interface 250 may provide an application program interface (API).

FIG. 2B is a block diagram of an example embodiment for ethnicity estimation engine 245 for training and utilizing a model to assign labels to a genotype dataset, according to one embodiment. The ethnicity estimation engine 245 trains and uses models to probabilistically determine the labels to which an input genotype sample corresponds. The ethnicity estimation engine 245 may be a computing system including one or more processors, one or more computer memories, and an interface for communicating through a network. In one example embodiment, the ethnicity estimation engine 245 includes a genealogy data store 252, a haploid MM Store 254, a reference panel sample store 256, a diploid HMM Store 258, an annotation store 262, a genetic data store 264, an inter-window HMM store 266, a range module 280, a confidence module 282, a polygon module 284, and a CNN store 260. The ethnicity estimation engine 245 can build and train a labeling model 290. The labeling model 290 includes various components (which may also be referred to as sub-models or modules) such as a haploid MM module 268, a diploid HMM module 270, an inter-window HMM module 272, a label assignment module 276, a phasing module 278 and a CNN module 274. In various embodiments, the labeling model 290 may include additional or fewer modules.

An online system, such as the ethnicity estimation engine 245, may maintain user data and genealogical data in the genealogy data store 252. The genealogy data store 252 stores user data for each user of the online system. The amount and type of data stored for each user in the genealogy data store 252 may vary based on the information provided by the corresponding user. Users may provide data via the user interface of a user device. The user interface may be a website or mobile application of the online system. For example, the user may be prompted in an element of a user interface to answer questions related to the user that can be processed to obtain genealogic and survey data. Examples of genealogical data include names (first, last, middle, suffixes), birth locations, date of birth, date of death, marriage information, kinships, family history, and the like. In some instances, family history can take the form of a pedigree of that individual (e.g., the recorded relationships in the family). The pedigree information associated with a user comprises one or more specified nodes. Each specified node in the pedigree represents either the individual or an ancestor of the individual corresponding to a stored DNA sample. Therefore, the pedigree includes the individual and ancestors who have passed down genetic material to the associated individual. The nodes in a pedigree may include personal information of the person (e.g., ancestor) represented by the node. For example, the personal information may include the geographical region in which the person was born. Other personal information may also take the form of various types of genealogical information.

Genealogical data may describe genetic connections among users of the online system. Genealogical data that are obtained from a public record source such as census records may be stored in the genealogy data store 252. Those records may include birth records, death records, marriage records, and census records. Genealogical data in the form of survey data include information about an individual's phenotypes, such as physical traits (e.g., height, hair, skin pigmentation, freckling, bitter taste, earlobe type, iris patterns, male pattern baldness, hair curl), wellness phenotypes (e.g., lactose tolerance, caffeine consumption, malaria resistance, norovirus resistance, muscle performance, alcohol flush), and personal preferences (e.g., likes and dislikes). The genealogy data store 252 may also include information inferred from the genetic data stored in the genetic data store 264 and information received from the individuals. For example, information related to which individuals are genetically related, how they are related, how many generations back they share common ancestors, percent IBD shared, which communities the individual is a part of, variants the individual carries, and the like.

Genealogical data may include data from one or more of a pedigree of an individual, the Ancestry World Tree system, a Social Security Death Index database, the World Family Tree system, a birth certificate database, a death certificate database, a marriage certificate database, an adoption database, a draft registration database, a veterans database, a military database, a property records database, a census database, a voter registration database, a phone database, an address database, a newspaper database, an immigration database, a family history records database, a local history records database, a business registration database, a motor vehicle database, and the like.

Genetic data store 264 maintains genetic datasets of individuals. Genetic data may contain whole or portions of individual's genome and corresponding metadata. The data stored in the genetic data store 264 may store one or more genetic datasets linked to a user. In various embodiments, the genetic data store 264 stores a pointer to a location associated with the Genealogy Data Store 252 associated with the individual. A genetic dataset may take different forms. In one embodiment, a genetic dataset may take the form of base pair sequence of the DNA sequence of an individual. A genetic dataset may include a whole genome of the individual (e.g., obtained from a whole-genome sequencing) or some parts of genetic loci. In another embodiment, a genetic dataset may take the form of sequences of target SNP sites and allele sites. The genetic dataset may be in the form of a diploid data and may be phased into two sets of haploid data. The diploid data may also be referred to as genotype data while the phased haploid data may be referred to as haplotype data.

In some embodiments, the ethnicity estimation engine 245 may operate in a training stage and a label assignment stage. The training stage may be performed once to train the labeling model 290 that includes sub-models. For example, a haploid MM for each window w stored in the haploid MM Store 254 may be trained to calculate the annotations stored in the annotation store 262 for each label $k$ and window w. The training stage is often based on more than a single particular input sample genotype dataset. For example, a collection of training samples may be used. After the training stage, the ethnicity estimation engine 245 may assign labels to an input sample genotype dataset $X$ during the label assignment stage. Assigning labels to the sample genotype dataset $X$ uses the haploid MMs and the annotations initialized during the training phase. In some embodiments, after the training stage for the labeling model 290 has been performed once, labels may be continuously assigned to different input genotype datasets. In other embodiments, after the labeling model 290 is initially trained, the ethnicity estimation engine 245 may continuously improve and update various components of the labeling model 290 by treating previously labeled input genotype datasets that were themselves labeled by the labeling model 290 as additional training samples.

The reference panel sample store 256 may include a collection of reference panel samples. Each reference panel sample may be a genetic dataset that is representative of a particular genetic community. For example, a Japanese reference panel sample may be representative of the genetic data of people of Japanese origin. Each ethnic origin may include more than one reference panel datasets. By comparing a window of genetic data of a target individual to different reference panel samples, the reference panel samples may be used to provide possible ethnic origin labels to the window of genetic data and may also assign a probability that the window of genetic data is inherited from a particular genetic community. This process of assigning labels and determining probabilities may be referred to as annotating.

The phasing module 278 phases diploid genetic dataset into a pair of haploid genetic datasets. An individual's haplotype may refer to a collection of alleles (e.g., a sequence of alleles) that are inherited from a parent. In one context, a haplotype may also refer to a collection of alleles that corresponds to a genetic segment. In other contexts, a haplotype may refer to a specific allele at a SNP site. For example, a sequence of haplotypes may refer to a sequence of alleles of an individual that are inherited from a parent.

Phasing may include a process of determining the assignment of alleles (particularly heterozygous alleles) to chromosomes. Owing to sequencing conditions and other constraints, a sequencing result often includes data regarding a pair of alleles at a given SNP site of a pair of chromosomes but may not be able to distinguish which allele belongs to which specific chromosome. The phasing module 278 uses a genotype phasing algorithm to assign one allele to a first chromosome and another allele to another chromosome. The genotype phasing algorithm may be developed based on an assumption of linkage disequilibrium (LD), which states that haplotype in the form of a sequence of alleles tends to cluster together. The phasing module 278 is configured to generate phased sequences that are also commonly observed in many other samples. Put differently, haplotype sequences of different individuals tend to cluster together. A haplotype-cluster model may be generated to determine the probability distribution of a haplotype that includes a sequence of alleles. The haplotype-cluster model may be trained based on labeled data that includes known phased haplotypes from a trio (parents and a child). A trio is used as training sample because the correct phasing of the child is almost certain by comparing the child's genotypes to the parent's genetic datasets. The haplotype-cluster model may be generated iteratively along with the phasing process with a large number of unphased genotype datasets.

By way of example, the phasing module 278 may use a directed acyclic graph model such as a hidden Markov model (HMM) to perform phasing of a target genotype dataset. The directed acyclic graph may include multiple levels, each level having multiple nodes representing different possibilities of haplotype clusters. An emission probability of a node, which may represent the probability of having a particular haplotype cluster given an observation of the genotypes may be determined based on the probability distribution of the haplotype-cluster model. A transition probability from one node to another may be initially assigned to a non-zero value and be adjusted as the directed acyclic graph model and the haplotype-cluster model are trained. Various paths are possible in traversing different levels of the directed acyclic graph model. The phasing module 278 determines a statistically likely path, such as the most probable path or a probable path that is at least more likely than 95% of other possible paths, based on the transition probabilities and the emission probabilities. A suitable dynamic programming algorithm such as the Viterbi algorithm may be used to determine the path. The determined path may represent the phasing result. U.S. Patent Application No. 15/591,099, entitled "Haplotype Phasing Models," filed on October 19, 2015, describes one possible embodiment of haplotype phasing.

The phasing module 278 may probabilistically separate the input sample genotype X into its constituent haplotypes based on the assigned labels. In one embodiment, a pair of labels for each window w is assigned based on the Viterbi path through the inter-window HMM. Phasing (i.e., separating the input sample genotype X into haplotypes) may be performed based on diploid HMMs 300 for each window w modified by the annotations $A_w$ for the assigned labels. For example, the diploid HMM for the input sample genotype X may be modified so that the probability of the diploid state $(u_1, u_2)$ in the window w is given by $A_w(u_1, p) \times A_w(u_2, q)$. The SNPs in the window w may be phased into the constituent haplotypes by determining the Viterbi path through the modified diploid HMM. In this way, the genome X may be phased so as to maximize the agreement with the label assignment. The haplotypes may also be combined across windows. For example, if the labels $(p, q)$ were assigned to window w and the labels $(p, q')$ were assigned to window w+1, then the sequence of alleles in the phased haplotype corresponding to label p in window w may be combined with the sequence of alleles in the phased haplotype corresponding to label p in window w+1. Similarly, the sequence of alleles in the phased haplotype corresponding to label q in window w may be combined with those of label q' in window w+1.

FIG. 2C illustrates differences between a non-admixed reference panel sample and an admixed reference panel sample. Reference panel samples may include two different types, depending on whether a population is unadmixed or admixed. For a non-admixed population, an entire genetic dataset of an individual may constitute a reference panel sample. For example, for population A, which is assumed to a non-admixed population, genetic datasets of individual 1, individual 2, and individual 3 may be three different reference panel samples that represent the genetic data of the population A. For population B, which is assumed to be an admixed population, a genetic dataset of an individual includes genetic segments that are inherited from different possible ethnic origins. For example, for a Hispanic population, the genetic dataset may include genetic segments of Native American origin, European origin, African origin, etc. For a particular ethnicity, various admixed individuals may have different genetic segments that are inherited from a particular ethnic origin. The online system may combine genetic segments of multiple admixed individuals to form a synthetic genetic dataset. For example, a reference panel sample for an admixed population may include a first genetic segment from a first admixed individual, a second genetic segment from a second admixed individual, etc. The first genetic segment and the second genetic segment are different segments.

The reference panel sample store 256 may include different reference panel samples for various ethnic origins of admixed individuals originated from the same geographical region. A synthetic reference panel formed by combining genetic segments from various individuals may be associated with a geographical region and an ethnic origin. For example, a synthetic genetic dataset representing Native American origin for a Hispanic population from Mexico may be associated with both Mexico (a geographical region) and Native American (an ethnic origin). The reference panel sample store 256 may include another synthetic genetic dataset representing European origin for the same Hispanic population from Mexico. This reference panel may be associated with Mexico and European. Likewise, a synthetic genetic dataset associated with Brazil (a geographical region) and European (ethnic origin) may also be a different reference panel. Put differently, for an admixed population from a particular geographical region, multiple reference panels representing different ethnic origins may be stored.

Haploid Markov Model

The haploid MM store 254 stores a plurality of haploid MMs (Markov Models), each haploid MM corresponding to a window w. The haploid MM module 268 builds the plurality of haploid MMs based on training data (e.g., sequenced haplotypes and/or phased haplotypes). In some embodiments, the haploid MMs may be received from another system (e.g., through a network). Each haploid MM is a probabilistic model of alleles in a respective window w. The haploid MM for a window w is a directed acyclic graph with a finite number of haploid states. Each directed edge between two haploid states in the haploid MM is referred to herein as a "transition" and corresponds to the value of an allele in a haplotype. Therefore, every possible haplotype (e.g., a sequence of alleles) in the window w corresponds to a path (i.e., sequence of haploid states) through the haploid MM corresponding to window w. The states in a haploid MM, the transitions between them, and the probabilities of those transitions are determined by the haploid MM module 268 based on the training data.

FIG. 3A illustrates an example of a haploid MM 300 for a window w, according to one embodiment. FIG. 3A illustrates the haploid MM for window w as a directed graph, where circles represent nodes with each node corresponding to a state, and arrows represent edges with each edge corresponding to a transition between a first state in a $d$-1th level to a second state in a $d$-th level. The haploid MM is divided into $D_w + 1$ levels (i.e., the haploid MM includes one more level than the number $D_w$ of SNPs in the window w). Each state in the model corresponds to some level $d \in \{0,..., D_w + 1\}$. Each level $d$ in the window w includes $h$ states. Each state $u$ in the haploid MM may be referenced by the combination of its level $d$ and an index $n$ (for $n \in \{0,...,h-1\}$), although states may be references with an alternate referencing scheme. In FIG. 3A, the index $n$ of each state $u$ is the integer with which the state is labeled. Herein, $u(w,d,n)$ references the $n$th state at level $d$ in window w.

Thus, the start state is $\mathbb{S}_w = u(w,0,0)$, state 202 is $u(w,2,2)$, and the end state is $\mathbb{E}_w = u(w,D_w,0)$.

A haploid MM 300 includes one start state $\mathbb{S}_w$ at level 0 and one end state at level $D_w$. Besides the end state $\mathbb{E}_w$ at level $D_w$ which is a terminal node, each state at level $d$ can include outgoing transitions to either one or two states at level $d+1$. The transition between a state at level $d$-1 to a second state in level $d$ corresponds to the $d$th allele in window w of a haplotype. In FIG. 3A, the allele value of a haplotype corresponding to the transition between two states is illustrated by the number (either 0 or 1) on the arrow between the states. For example, the transition from the start state to $u(w,1,0)$ (i.e., the state at level 1 with index number $n = 0$) corresponds to an allele of 0 at the first SNP position in window w $and$ the transition from the start state $\mathbb{S}_w$ to $u(w,1,1)$ (i.e., the state at level 1 with index number $n = 1$) may correspond to an allele of 1 at that SNP position. As indicated by FIG. 3A, in this example, the transition probability between the start state $\mathbb{S}_w$ and $u(w,1,0)$ is 0.56 and the transition probability between and $u(w,1,1)$ is 1 - 0.56 = 0.44.

In the haploid MM 300, the transition function $t(u,a)$ describes the transition of a haploid state $u$ in a $d$-1th level to an allele value $a$ in the $d$-th level, where the allele value $a$ may take a binary value (e.g., $a \in \{0,1\}$). For example, in FIG. 3A, $t(u(w,2,0),0)$ describes the transition from $u(w,2,0)$ to $u(w,3,0)$ because $u(w,3,0)$ is the next state that has the allele value 0. Likewise, $t(u(w,2,0),1)$ describes the transition to haploid state $u(w,3,1)$ because $u(w,3,1)$ is the next state that has the allele value 1. When a haploid state $u$ at level $d$-1 transitions to two distinct states (i.e., when $t(u,0) \neq t(u,1)$), each of the transitions is mapped to the $d$th allele in the window w. Herein, $\rho(u,a)$ refers to the transition probability that state $u$ at level $d$-1 transition to next state at the $d$-th SNP that has an allele that takes the value of $a$. For example, an edge 204, which represents $u(w,2,1)$ transitioning to the next state that has an allele value of 0, corresponds to the transition probability $p(u(w,2,1),0) = 0.9$. Likewise, an edge 206 corresponds to the transitional probability $p(u(w,2,1),1) = 0.1$. If the state $u$ transitions to only one state $v$ at level $d$, then the haploid MM may still include a probability distribution for the $d$-th allele even though the state transition is deterministic. For example, as illustrated in FIG. 3A, the transition from state $u(w,2,2)$ to state $u(w,3,4)$ may associate a probability of 0.75 with allele 0 at the third SNP in the window w and a probability of 0.25 for allele 1 at the third SNP in the window w.

Each path through the haploid MM 300 corresponds to one or more possible sequences of alleles (for example, that may occur in the input sample genotype dataset X). The probability of a sequence of alleles is given by the product of the corresponding allele probabilities in the corresponding path. For example, a path that includes the sequence of state ($\mathbb{S}_w$, $u(w,1,1)$, $u(w,2,1)$, $u(w,3,3)$) corresponds to the sequence of alleles (1,1,1) which has a probability of $\rho(\mathbb{S}_w,1) \times \rho(u(w,1,1),1) \times \rho(u(w,2,1),1) = 0.022$. The possible haplotypes (or, equivalently, every possible sequence of alleles) correspond to different paths in the haploid MM. Each path corresponding to a possible haplotype begins at the start state , includes exactly one state for each level $d$, and ends at the end state .

Diploid Hidden Markov Model

Returning to FIG. 2B, the diploid HMM Store 258 stores a plurality of diploid HMMs (hidden Markov Models). Each diploid HMM corresponds to each window $w$. The diploid HMM module 270 may build these diploid HMMs based on the haploid MMs stored in the haploid MM Store 254. Each diploid state in the diploid HMM for window w corresponds to an ordered pair of haploid states (i.e., one haploid state for each of the two haplotypes that constitutes a genome) in the haploid MM 300 for window $w$. Thus each diploid state $(u_1, u_2)$ in the diploid HMM at level $d$ corresponds to the haploid states $u_1$ and $u_2$, where $u_1$ and $u_2$ are from level $d$. For example, the start state of the diploid HMM for window w is $(u(w,0,0), u(w,0,0)) = (\mathbb{S}_w, \mathbb{S}_w)$. In some embodiments, the haploid states $u_1$ and $u_2$ are phased, meaning $u_1$ is used to represent a first parent haplotype such as a paternal haplotype while $u_2$ is used to represent a second parent haplotype such as a maternal haplotype, or vice versa.

In some embodiments, the diploid HMM Store 258 stores a full HMM for each window $w$. A full diploid HMM for window $w$ includes, for a level $d$, a diploid state for every ordered pair of haploid states in the haploid MM 300 at level $d$. Full diploid HMMs may be calculated during a training stage. The diploid HMM store may also include diploid HMMs that correspond to particular genotype datasets. The diploid HMM for a particular genotype dataset $G$ (e.g., an input sample genotype dataset $X$ or a reference panel sample genome for a label $k$) in window $w$ may include all the possible diploid states that are compatible with the genotype dataset $G$ and the possible transitions for genotype dataset $G$. Diploid HMMs may be computed for the input sample genotype dataset $X$ by the diploid HMM module 270 during a label assignment stage. Diploid HMMs may be also be computed for the reference panel sample genomes stored in the reference panel sample store 256 during the training stage when calculating the annotations in the annotation store 262. In general, the diploid HMM for window $w$ for a genotype dataset $G$ sometimes includes fewer states than the full diploid HMM for window $w$, because many diploid states in the full diploid HMM may not be compatible with the genotype $G$.

In some embodiments, a diploid HMM for a genotype dataset $G$ for a window $w$ is computed based on the full diploid HMM for the window $w$. In alternate embodiments, the diploid HMM module 270 does not build from full diploid HMMs and no full diploid HMMs are stored in the diploid HMM Store 258. Instead, the diploid HMM module may build diploid HMMs for genotype datasets for each window $w$ based on the corresponding haploid MM for window $w$.

FIG. 3B is an example of a diploid HMM 301 for a window w, according to an embodiment. The diploid HMM illustrated in FIG. 3B is a fully-instantiated diploid HMM. For this reason, the number of diploid states at each level $d$ for window $w$ is equal to the square of the number of states in the corresponding haploid MM 300 at level $d$ (i.e., $h^2$). For a genotype sequence made up of haplotypes that correspond to a diploid state $(u_1, u_2)$ at level $d$-1, the probability that the $d$-th alleles in the window w is the ordered pair $(a_1, a_2)$ equals $\rho(u_1, a_1) \times \rho(u_2, a_2)$. The number of possible transitions from a diploid state $(u_1, u_2)$ to another state is equal to the number of possible transitions from $u_1$ in the haploid MM to a next state multiplied by the number of possible transitions from $u_2$ to a next state.

FIG. 3B depicts an example diploid HMM 301 that corresponds to the example haploid MM 300 depicted in FIG. 3A. In FIG. 3B, each node represents a diploid state in the diploid HMM that is labeled with a pair of index numbers $(n,m)$ corresponding to the indices of the corresponding pair of haploid states in the haploid MM 300. For example, the diploid state labeled (1,2) at level 2 in FIG. 3B represents the diploid state $(u(w,2,1),u(w,2,2))$ where haploid states $u(w,2,1)$ and $u(w,2,2)$ are from the haploid MM 300 of FIG. 3A. In some embodiments, the diploid states are phased so that the order of the two haploid states in a pair of diploid state represents the phase of the haploid states. For example, the diploid state labeled (1,2) represents that the first parent state is in the haploid state 1 while the second parent state is in the haploid state 2 while the diploid state labeled (2,1) represents that the first parent state is in the haploid state 2 while the second parent state is in the haploid state 1, or vice versa if the first state is denoted as the maternal state.

Because every genotype dataset corresponds to two haplotypes, each phased genotype dataset corresponds to a single path through the diploid HMM 301 for window $w$. However, because the SNPs in unphased genotype datasets do not associate alleles with particular haplotypes, the exact path through the diploid HMM that a genotype dataset traverses may be ambiguous as the genotype dataset will likely include a number of heterozygous SNPs and possibly missing data for SNPs as well. For example, the sequence of unordered allele pairs ((0,1),(0,1)) corresponds to four distinct paths through the first three levels of the example diploid HMM for window w such as the sequence of diploid states $((\mathbb{S}_w, \mathbb{S}_w),(u(w,1,0),u(w,1,1)),(u(w,2,1),u(w,2,2)))$. In addition, chromosome crossover may occur during meiosis.

For various reasons, the phasing of a genotype dataset is not a deterministic process and, thus, there may be errors in phasing and determining a pair of haplotype sequence datasets from a genotype dataset.

The diploid HMM 301 may be used to generate a pair of phased haplotype datasets of an input genotype dataset for each window w. The input genotype dataset can be used with other training datasets to iteratively build the diploid HMM 301 for a predetermined number of iterations or until the diploid HMM 301 converges. For example, the diploid HMM 301 is initially trained with the reference panel samples obtained from the reference panel store 115. The reference panel samples may be unadmixed datasets or synthetic datasets for admixed populations. A different diploid HMM 301 may be computed and trained for each pair of labels using the reference panels associated with the pair of labels. The input genotype dataset may then be used as an input of the trained diploid HMM 301 to determine the Viterbi path of the diploid HMM 301. The Viterbi path may represent a likely outcome of a pair of phased haplotype datasets. The phased haplotype dataset may then be used as one of the training samples to improve the diploid HMM 301. This iteration may be repeated multiple times to improve the Viterbi path calculation and the phasing of the input genotype dataset. For more information on the phasing of an input genotype dataset to generate a pair of phased haplotype datasets, U.S. Patent Application Publication No. 2017/0262577 published September 14, 2017, entitled "Haplotype Phasing Models," is mentioned herein for all purposes. For more information on methods and predicting labels using HMM, U.S. Patent Application Publication No. 2020/0082903A1 published March 12, 2020, entitled "Global Ancestry Determination System," and U.S. Patent Application Publication No. 2017/0017752A1 published January 19, 2017, entitled "Local Genetic Ethnicity Determination System," is mentioned herein for all purposes.

### Inter Window Hidden Markov Model

After a pair of phased haplotype datasets are generated from an input sample genotype dataset X, the ethnicity estimation engine 245 assigns labels to the input genotype dataset *X* by using and constructing an inter-window hidden Markov model (inter-window HMM). The genetic data store 264 stores one or more pairs of phased haplotype datasets. The ethnicity estimation engine 245 may assign labels to the input sample genotype dataset X based on the pair of phased haplotype datasets. The inter-window HMM store 266 stores an inter-window HMM corresponding to the input sample genotype dataset X that is used to determine the labels. The inter-window HMM is computed or built by the inter-window HMM module 272. The inter-window HMM includes states for each window *w.*

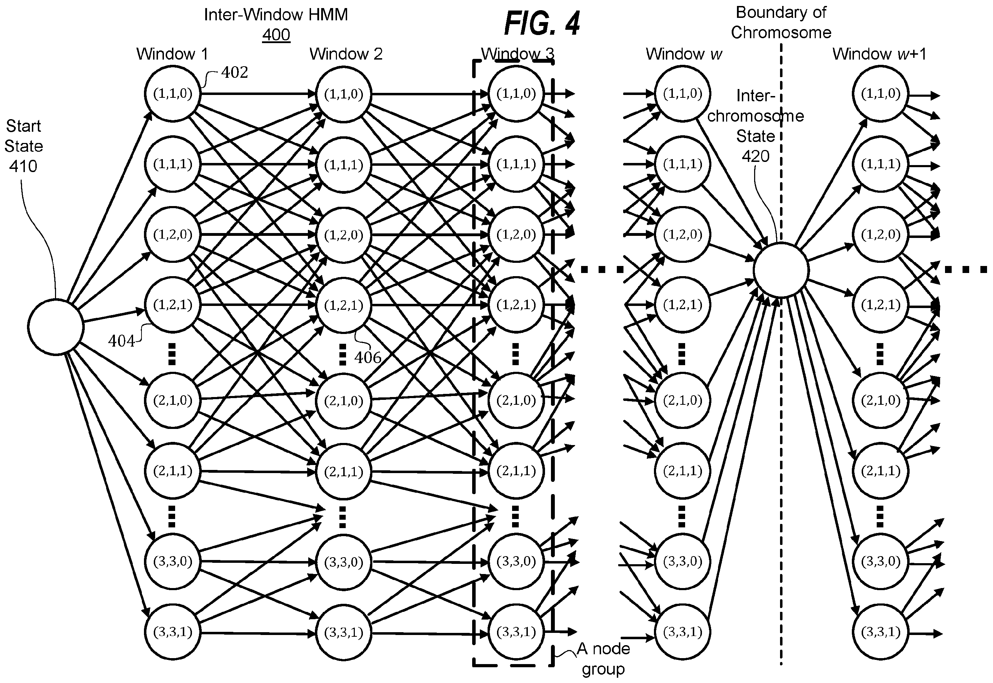

FIG. 4 illustrates a simplified example of an inter-window HMM 400, according to an embodiment. The inter-window HMM 400 may be a directed (e.g., in the direction from left to right as shown in FIG. 4) acyclic graph that includes a plurality of node groups. The graph representing the inter-window HMM 400 may also be referred to as a trellis. Graphically, each node group in the trellis may also be referred to as a level, a slot, a graph window, or a layer. Each node group represents a window w that corresponds to a genetic segment such as a set of SNPs. A plurality of nodes (represented by the circles in FIG. 4) are arranged in each node group. Each node represents a possible state of the window w. Each node is associated with an emission probability representing a likelihood of the window is observed as having a particular pair of phased haplotype datasets given the window is having the hidden state (i.e., the window is assigned with a particular pair of labels). In other words, the particular pair of phased haplotype datasets may be an observation in a hidden Markov model while the state that is labeled may be the "hidden" state of the hidden Markov model because the labels are not apparent given only the genotype dataset or the phased haplotype dataset. The inter-window HMM 400 also includes a plurality of edges. Each edge connects a first node of a first node group to a second node of a second node group. Each edge represents a transition from the first node of the first node group to the second node of the second node group. Each edge is associated with a transition probability that represents a likelihood of transition from the first node to the second node. The determination of the transition probabilities will be discussed in further details below in association with FIG. 9.

A state (represented by a node) in the inter-window HMM 400 includes three different labels. In the particular embodiment shown in FIG. 4, the three labels are orderly presented as a first parent label, a second parent label, and a switch label that represents a switch of the order between the first parent label and the second parent label in the particular window, where the switching may be associated with phasing errors. While the order of presentation in the embodiment shown in FIG. 4 is the first parent label, the second parent label, and the switch label, other orders of presentation are also possible.

Each of the three labels in a state is represented by an integer value. For example, both the first parent label and the second parent label are selected from a set of K possible labels. A label is a classification of genetic data. For example, one possible way to classify genetic data is by ethnic origins of the individual, although other ways to classify genetic data are possible and are not necessarily based on or related to ethnic origins. If ethnic origins are used as classification, the set of K possible labels may be African, Asian, European, etc. or be German, Korean, Mexican, etc., depending on the granularity of the classification. A particular integral value represents one of the labels. For example, 1 may represent European while 2 may represent Asian.

The third label of a node, which is the switch label, may take a binary value (e.g., 1 or 0). The first binary value (e.g.,

1) may represent that there is a switching of order of the first parent label and the second parent label while the second binary value (e.g., 0) may represent that there is no switching of order. A switch label represents a switching of order of the first parent label and the second parent label. In other words, a switch label represents that, for a particular state, the order of the first parent label and second parent label in the HMM is switched compared to the actual labels in the sample. Using the examples discussed in this paragraph as an illustration, the first node 402 of Window 1 in FIG. 4, which takes the values (1, 1, 0), may represent the state that Window 1 is labeled as European for both first parent label and second parent label and there is no switching of order between the two labels.

Likewise, the fourth node 404 of Window 1 in FIG. 4, which takes the values of (1, 2, 1), may represent the state that Window 1 is labeled as European for first parent label and Asian for second parent label but there is a switching of order between the two labels. In other words, due to one or more possible, but unobserved reasons such as a phasing error, the fourth node 404 in fact represents that Window 1 has Asian as first parent label and European for second parent label.

Using node 402 as an example to explain the concept of emission probability in the inter-window HMM 400, the emission probabilities here represent the likelihoods that Window 1 is observed in the sample genotype dataset to have a particular pair of phased haplotype datasets given the Window 1 should be labeled as having European origin for both first parent ancestry and second parent ancestry. Likewise, the transition probability from the node 402 to the node 406 represents the likelihood that a first segment of SNPs (corresponding to Window 1), which should be labeled as having European origin for both first and second parent ancestries, transitions to a second segment of SNPs (corresponding to Window 2) that should be labeled as having European origin for the first parent ancestry and European origin for the second parent ancestry, but there is a switching of first parent label and second parent label.

The plurality of nodes in each node group represents permutations of different possible first parent labels, second parent labels, and switch labels that can be assigned to a window. For each window, the inter-window HMM 400 may include a set of states corresponding to every ordered set of labels. Hence, the total number of states ($T$) can be $K*K*2$ (first parent labels $K$ * second parent labels $K$ * binary switch labels) for each window. For the particular embodiment shown in FIG. 4, there are three possible values of classification labels (i.e., $K = 3$) and the switch label takes the value of either 1 or 0. Hence, there are $3*3*2 = 18$ possible states (i.e., $T = 18$). For simplicity, only some of the states are shown in FIG. 4 for each window. The states for a window $w$ are denoted as $U_w(p,q,z)$ where $p$ is the value of the first parent label (e.g., $p \in (1,2, ..., K)$), $q$ is the value of the second parent label (e.g., $q \in (1,2, ..., K)$), and $z$ is the value of the switch label (e.g., $z \in (0,1)$). In this way, the set of labels $(p,q,z)$ uniquely refers to each of the possible states $T$. Although FIG. 4 depicts $K=3$ labels, the number of labels $K$ can be any natural integers.

The inter-window HMM 400 is a directional graph that represents a transition from a start state to an end state (not shown in FIG. 4) through a plurality of node groups that represent a plurality of windows. The start state 410 transitions to one of the $T$ possible states of window 1 as illustrated by the arrows between the start state 410 and the respective $T$ states of window 1. Each state in window 1 may transition to one of the possible states in window 2. A state $U_w(p,q,z)$ in window $w$ may transition to a state $U_{w+1}(p',q',z')$ in window $w+1$. The chromosome that corresponds to the window $w$ is denoted as $C(w)$ while the chromosome that corresponds to the window w+1 is denoted as $C(w+1)$. If the window $w$ and the window $w$ +1 correspond to the same chromosome (i.e., $C(w) = C(w+1)$), then a state $U_w(p,q,z)$ may be more likely to transition to a state $U_{w+1}(p',q',z')$ in window $w+1$ that corresponds to the same pair of labels (i.e., $(p',q') = (p,q)$) without switching than to a state in window $w+1$ that corresponds to a different pair of labels or to a state in window $w+1$ that corresponds to a switching of labels. This is because it is biologically unlikely that the sequences of SNPs in adjacent windows will correspond to different labels (e.g., correspond to different ancestral origin groups).

In some embodiments, the transition probability $P(U_w(p,q,z), U_{w+1}(p',q',z'))$ from a state $U_w(p,q,z)$ to a state $U_{w+1}(p',q',z')$ is given by equation (1) below:

$$P(U_w(p,q,z) \rightarrow U_{w+1}(p',q',z')) =$$

$$
\begin{cases}
\dfrac{\pi_{p'}^m \times \pi_{q'}^f}{2} & \text{if } C(w) \neq C(w+1) \\[2mm]
(1-\tau^m) \times (1-\tau^f) \times (1-\tau^z) & \text{if } C(w) = C(w+1), p = p', q = q', z = z' \\[2mm]
(1-\tau^m) \times (1-\tau^f) \times \tau^z & \text{if } C(w) = C(w+1), p = p', q = q', z \neq z' \\[2mm]
\tau^m \times (1-\tau^f) \times (1-\tau^z) \times \dfrac{\pi_{p'}^m}{\sum_1^{p'-1} \pi_k^m + \sum_{p'+1}^K \pi_k^m} & \text{if } C(w) = C(w+1), p \neq p', q = q', z = z' \\[2mm]
\tau^m \times (1-\tau^f) \times \tau^z \times \dfrac{\pi_{p'}^m}{\sum_1^{p'-1} \pi_k^m + \sum_{p'+1}^K \pi_k^m} & \text{if } C(w) = C(w+1), p \neq p', q = q', z \neq z' \\[2mm]
(1-\tau^m) \times \tau^f \times (1-\tau^z) \times \dfrac{\pi_{q'}^m}{\sum_1^{q'-1} \pi_k^f + \sum_{q'+1}^K \pi_k^f} & \text{if } C(w) = C(w+1), p = p', q \neq q', z = z' \\[2mm]
(1-\tau^m) \times \tau^f \times \tau^z \times \dfrac{\pi_{q'}^m}{\sum_1^{q'-1} \pi_k^f + \sum_{q'+1}^K \pi_k^f} & \text{if } C(w) = C(w+1), p = p', q \neq q', z \neq z' \\[2mm]
0 & \text{if } C(w) = C(w+1), p \neq p', q \neq q'.
\end{cases}
$$

The symbol $\pi_k^m$ represents the label probability distribution of first parent label $k$ over $K$ different labels while $\pi_k^f$ represents the label probability distribution of second parent label $k$ over $K$ different labels. In some embodiments the label probability distributions may each correspond to a genome wide distribution, but in other embodiments the distributions may correspond to a portion of the genome. In some cases, the label probabilities over all different labels sum to unity (i.e., $\sum_{k \in K} \pi_k^m = \sum_{k \in K} \pi_k^f = 1$). The label probability distributions $\pi_k^m$ and $\pi_k^f$ indicates the preference of parent 1 and parent 2, respectively, for $K$ different labels. For example, $\pi_{p'}^m$ is the probability of first parent label of window $w$+1 taking the value k = $p'$ over other possible values of labels $K$. C($w$) = C($w$+1) represents that the two windows correspond to the same chromosome. The label change probability $\tau^m$ represents the probability that first parent label will transition to a different label from window w to window $w$+1 (e.g., window w has a label of European while window w+1 has a label of Asian). In the embodiment that uses the equation above, the change of label depends on label probability $\pi_k^m$ and $\pi_f^m$. The label change probability $\tau^f$ represents the probability that second parent label will transition to a different label from window w to window w+1. The label switch probability $\tau^z$ represents the probability that the order of first parent label and the second parent label is switched (i.e. the state will transition to the opposite *z* assignment between two windows.)

Hence, in the above equation, the first scenario represents that two windows are located in different chromosomes and the transition probability $P(U_w(p,q,z), U_{w+1}(p',q',z'))$ is equal to the first parent label probability of $k$ = $p'$ times the second parent label probability of $k$ = $q'$ divided by 2. The second scenario represents that the two windows are located in the same chromosome and there is no change in label or switch of label order. The transition probability in this scenario is equal to one minus the first parent label change probability $\tau^m$ (because the label either change or does not change) times one minus the second parent label change probability $\tau^f$ times one minus label switch probability $\tau^z$. Other scenarios are modeled similarly in the equation above.

The values of label probabilities ( $\pi_k^m$ and $\pi_k^f$ ), label change probabilities ($\tau^m$ and $\tau^f$), and the label switch probability ($\tau^z$) are determined by the training of the inter-window HMM 400 based on a set of training data and, in some embodiments, additionally with the pair of haplotype datasets derived from an input sample genotype dataset $X$. The values of label probabilities ( $\pi_k^m$ and $\pi_k^f$ ) of different $k$ may be represented in a vector form (also referred to as label probability vector). In some embodiments, the values of the label probability vector and the label change probabilities are calculated with a Baum-Welch algorithm. In some embodiments, it may be assumed that a transition from a state $U_w(p,q,z)$ to another state $U_{w+1}(p',q',z')$ without any of the same labels $p$, $q$ (i.e., both values of first parent label and second parent label change in a transition) are impossible. Hence, the transition probability for the last scenario in the equation above is zero in some embodiments. By omitting a transition for these low-probability transitions, the complexity of the inter-window HMM 400 may be reduced, thereby producing significant savings in time and computer processing requirements needed to determine labels.

If the window $w+1$ corresponds to a different chromosome than window w, then the state $U_w(p,q,z)$ may transition to an inter-chromosome state 420, which, in turn, transitions to a state $U_{w+1}(p',q',z')$ in the next window $w+1$. Thus, if the window $w+1$ corresponds to a different chromosome than window w, the state $U_w(p,q,z)$ may transition to a state $U_{w+1}(p',q',z')$ with a probability that is independent of the state $U_w(p,q,z)$ at window w (i.e., independent of $(p,q)$) because of the intervening inter-chromosome state 420.

If window w is the final window (i.e., $w = W$), then the state $U_w(p,q,z)$ in the window w transitions to an end state (not shown in FIG. 4). Each state $U_w(p,q,z)$ in window w transitions to either a state $U_{w+1}(p',q',z')$ in window $w+1$, an inter-chromosome state 420, or an end state. FIG. 4 illustrates the possible outgoing transitions for each state $U_w(p,q,z)$ with arrows. For example, in window 2 (and in all windows w in which the window $w+1$ is on the same chromosome), the state 406 $U_2(1,2,1)$ may transition to the states $U_3(1,1,0)$, $U_3(1,1,1)$, $U_3(1,2,0)$, $U_3(1,2,1)$,, etc. However, the state 406 $U_2(1,2,1)$ may not transition to state $U_3(3,3,0)$ because of both the first parent label and second parent label change in the transition. As such, no arrow connects the state 406 $U_2(1,2,1)$ to the state $U_3(3,3,0)$ in FIG. 4.

Determine Emission Probabilities Using Convolutional Neural Network

In FIG. 4, each node (representing a state of a window) is associated with an emission probability that represents a likelihood of the window is observed as having a particular pair of phased haplotype datasets given the window is in the hidden state represented by the node. In one embodiment, the emission probabilities for building an inter-window HMM may be determined by using machine learning models such as deep neural networks including convolutional neural network (CNN), recurrent neural networks (RNN) and long short-term memory networks (LSTM). Deep neural networks transform input data into abstract feature representations by connecting a series of nodes through nonlinear transformations, where each feature representation may be a nonlinear combination of the input data. A neural network may be associated with a set of parameters that are continuously updated based on prediction errors. The neural network may stop updating when the prediction error or the change in prediction error is within a certain threshold.

In various embodiments, the training techniques for a machine learning model may be supervised, semi-supervised, or unsupervised. In supervised learning, the machine learning models may be trained with a set of training samples that are labeled. For example, for a machine learning model trained to classify ethnicity, the training samples may be different genetic samples of individuals labeled with the known ethnicity. In some cases, those training samples with known ethnicity are referred to as reference panels. The identification of those reference panels is discussed in association with the reference panel sample store 240. The labels for each training sample may be binary or multi-class. In some cases, an unsupervised learning technique may be used, where the samples used in training are not labeled. Various unsupervised learning technique such as clustering may be used. In some cases, the training may be semi-supervised with training set having a mix of labeled samples and unlabeled samples.

A machine learning model may be associated with an objective function, which generates a metric value that describes the objective goal of the training process. For example, the training goal may be to reduce the error rate of the model in generating predictions. In such a case, the objective function may monitor the error rate of the machine learning model. For example, the objective function of the machine learning algorithm may be the training error in predicting ethnicity labels for individuals given haplotype datasets. Such an objective function may be called a loss function. Other forms of objective functions may also be used, particularly for unsupervised learning models whose error rates are not easily determined due to the lack of labels. In various embodiments, the error rate may be measured as cross-entropy loss, L1 loss (e.g., the sum of absolute differences between the predicted values and the actual value), L2 loss (e.g., the sum of squared distances).

In one embodiment, emission probabilities for a window in the inter-window HMM may be determined by a CNN model. Briefly referring back to FIG. 2B, the CNN store 260 stores one or more CNN models and parameters associated with the CNN models for each window. The CNN module 274 may train one or more CNN models for determining emission probabilities for each window. Each window may be associated with a different trained CNN model. The CNN models may be trained based on genotype or haplotype datasets from a reference panel, where a reference panel contain datasets associated with known members from an ethnic population. The different CNN models and their respective parameters are stored in the CNN store 260.

FIG. 5 is an example structure of a convolutional neural network (CNN) for calculating emission probabilities for a given window, according to an embodiment. The CNN 500 may receive an input 510 and generate an output 520. To train the CNN 500 for a particular window, the input 510 may be haplotype datasets phased from genotype datasets for the particular window, where the haplotype datasets include a plurality of SNPs for the window. The genotype datasets are associated with individuals from a reference panel with known ethnic groups and therefore each genotype data sample has a known label indicating the ethnic group that the individual belongs to.

In one embodiment, the input 510 may include additional training data generated through data augmentation, which is a technique used to increase the amount of training data by generating synthetic data based on existing data. The synthetic data may be generated by recombining existing haplotype datasets from the reference panel. Haplotype datasets may be selected from the reference panel and recombined to generate additional training data. For example, to generate a new data sample, a number of N haplotype datasets may be selected to contribute to the recombinant. The number N may be determined based on a Poisson distribution. A number of breakpoints may be randomly generated on the selected haplotype datasets and each haplotype dataset maybe segmented into a plurality of segments based on the breakpoints. Then, a new data sample may be generated by combining different segments from the selected haplotype datasets and the new data sample may be used as additional training data for the CNN 500.

The CNN 500 may include different types of layers, such as convolutional layers 530, pooling layers 540, full connected layers 560, and custom layers 570. A convolutional layer 530 convolves the input of the layer with one or more kernels to generate abstract features representations from the input 510. Kernels in a CNN model may be used to extract features from the input data by performing operations (e.g. dot product) with sub-regions of input data. Kernels may be a matrix or an array with different sizes (e.g. 3x3 matrix or array of length 5). In one embodiment, because the input data is a one-dimensional data sequence, the kernel for the CNN 500 may be a sliding window of one dimension that moves in a direction along the haplotype dataset. Each convolutional layer 530 may output a convolution result which is further passed through an activation function, which further process the convolution results, such as incorporating nonlinearity to the convolution results.

A convolutional layer 530 may be followed by a pooling layer 540 that selects the maximum value (max pooling) or average value (average pooling) from the portion of the input covered by the kernel size. The pooling layer 540 reduces the spatial size of the extracted features. The convolutional layer 530 and the pooling layer 540 may be followed by multiple fully connected layers 560 that contain nodes (represented by squares in FIG. 5) connected to each other. The fully connected layers 560 may be used for classification and feature detection. In one embodiment, one or more custom layers 570 may also be presented for the generation of a specific format of output 520. For example, a custom layer 570 may include various layers such as softmax layers, flatten layers and dropout layers. A softmax layer may transfer a vector with a plurality of real values to a vector with a plurality of probabilities that sum to 1, where the vector with probabilities corresponds to the predicted probabilities that the individual is predicted to belong to each ethnic group. A flatten layer may convert multidimensional input to a one-dimensional vector and a dropout layer may randomly drop nodes to prevent overfitting. In the embodiment illustrated in FIG. 5, the custom layers are placed after the convolutional layers 520 and the pooling layers 540. For example, the softmax layer may be the final layer for generating the final output probabilities. In some embodiments, one or more custom layers 570 may be before the fully connected layers 560 or before one or more of the convolutional layers 530 and the pooling layers 540.

The order of layers and the number of layers of the CNN 500 in FIG. 5 is for example only. In various embodiments, a CNN 500 includes one or more convolutional layer 530 but may or may not include any pooling layer 540. A CNN 500 may include multiple convolutional layers 530, each followed by a pooling layer 540, as illustrated in FIG. 5. If a pooling layer 540 is present, not all convolutional layers 530 are always followed by a pooling layer 540. For each convolutional layer 530, the sizes of kernels (e.g., 3x1, 5x1, 7x1, etc.) and the numbers of kernels allowed to be learned may be different from other convolutional layers 530.

A machine learning model may include certain layers, nodes, kernels and/or coefficients. Training of a neural network, such as the CNN 500, may include forward propagation and backpropagation. Each layer in a neural network may include one or more nodes, which may be fully or partially connected to other nodes in adjacent layers. In forward propagation, the neural network performs the computation in the forward direction based on outputs of a preceding layer. The operation of a node may be defined by one or more functions. The functions that define the operation of a node may include various computation operations such as convolution of data with one or more kernels, pooling, recurrent loop in RNN, various gates in LSTM, etc. The functions may also include an activation function that adjusts the weight of the output of the node. Nodes in different layers may be associated with different functions.

Each of the functions in the neural network may be associated with different coefficients (e.g. weights and kernel coefficients) that are adjustable during training. In addition, some of the nodes in a neural network may also be associated with an activation function that decides the weight of the output of the node in forward propagation. Common activation functions may include step functions, linear functions, sigmoid functions, hyperbolic tangent functions (tanh), and rectified linear unit functions (ReLU). After an input is provided into the neural network and passes through a neural network in the forward direction, the results may be compared to the training labels or other values in the training set to determine the neural network's performance. The process of prediction may be repeated for other training data in the training sets to compute the value of the objective function in a particular training round. In turn, the neural network performs backpropagation by using gradient descent such as stochastic gradient descent (SGD) to adjust the coefficients in various functions to improve the value of the objective function.

Multiple rounds of forward propagation and backpropagation may be performed. Training may be completed when the objective function has become sufficiently stable (e.g., the machine learning model has converged) or after a predetermined number of rounds for a particular set of training samples. The trained machine learning model can be used for performing prediction, object detection, image segmentation, or another suitable task for which the model is trained.

For example, the CNN 500 may be trained with a plurality of haplotype datasets associated with individuals from the reference panel and each of the haplotype dataset is associated with a known ethnic label. The CNN 500 may perform one iteration of forward propagation by passing the input dataset through each layer and finally generates an output 520, which is a prediction of emission probabilities for each haplotype dataset, and the ethnic origin associated with the highest emission probability is predicted to be the ethnic label for a haplotype dataset. The CNN 500 may in turn compute an error based on an objective function by comparing the predicted results with the true labels. The error may be passed backwards through backpropagation to determine an adjustment to parameters of the CNN 500 such that the error is reduced. A forward pass and a backward pass may be referred to as an iteration. The CNN 500 may repeat the process for multiple iterations until a desired goal is achieved, where a goal may be that the error is within a certain threshold or that the change in error between consecutive iterations is within a certain threshold. The CNN 500 may also stop iterating if a predetermined number of iterations is reached. Responsive to a desired goal is achieved, the CNN 500 may stop iterating and the CNN module 274 may save the model and the parameters to the CNN store 260.

In one embodiment, the CNN module 274 may hold out a portion of the total data (e.g. 10% of the original data) as a validation dataset and another portion (e.g. 5% of the original data) as test data. The validation data may be used to make predictions based on the parameters trained by the training dataset. Based on the prediction results using the validation dataset, the CNN module 274 may further finetune hyperparameters (such as number of layers) in the CNN 500. The test data is used to access the performance of the model CNN 500 after training and fine tuning.

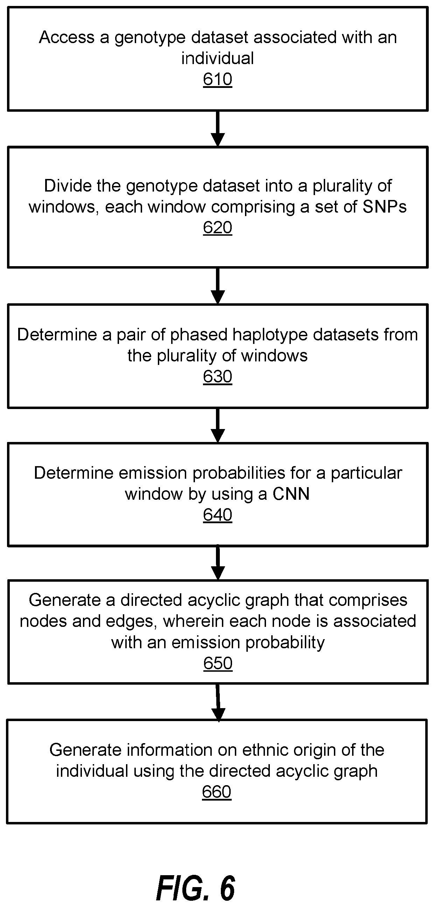

FIG. 6 illustrates a process of determining emission probabilities using CNN, according to one embodiment. The CNN module 274 may predict emission probabilities for a target individual based on a genotype dataset associated with the target individual. The CNN module 274 may access 610 a genotype dataset associated with an individual and may divide 620 the genotype dataset into a plurality of windows, each window comprising a set of SNPs. Using phasing algorithms, a pair of haplotype datasets are determined 630 based on the plurality of windows of genotype datasets. Using one or more of the trained CNN models stored in the CNN store 260, the CNN module 274 may determine 640 emission probabilities for a particular window. The ethnicity estimation engine 245 may generate a directed acyclic graph that comprises a plurality of node groups contains nodes, and a plurality of edges. Each node group corresponds to a window and contain nodes that are each associated with emission probabilities. Based on the directed acyclic graph, the ethnicity determination engine 245 may generate information on the ethnic origin of the individual.

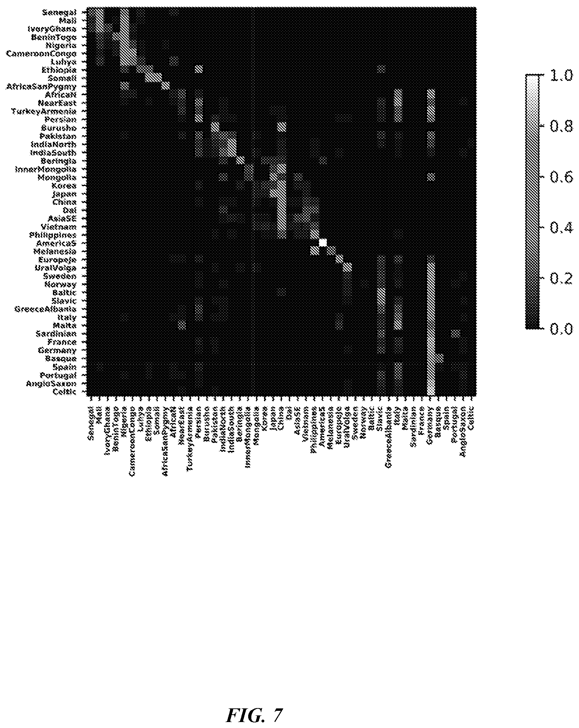

FIG. 7 illustrates a confusion matrix displaying predicted results by using CNN models before applying the inter-window HMM. The illustrated confusion matrix is based on CNN models that are built for one of the plurality of windows and a predicted label (i.e. ethnic group) is determined based on the highest emission probability associated with one of the plurality of labels in the window. Each row of the confusion matrix represents haplotypes from a particular ethnicity and each column represents the predicted percentage of individuals assigned to each ethnicity. For example, the last row of the confusion matrix is labeled as Celtic, and therefore the last row represents the predicted labels for the haplotype data sets that are known to come from a Celtic origin. The different shades of each small square for the last row indicate different percentages that the Celtic haplotype datasets are predicted to come from other ethnic origins. As indicated by the labeled scale on the right, the lighter the color is, the higher the predicted percentage is. As presented in FIG. 7, the diagonal area of the confusion matrix is of the lightest shade, which indicates the CNN models achieves a desirable result.

Determination of Emission Probability based on annotations

In one embodiment, the determination of the emission probability may also be based on genotype data of different reference panels and the input genotype dataset X through one or more intermediate steps that may include determinations of annotations, annotation products, and label pair probabilities. The details of the determination of the emission probability based on annotations is discussed below.

Returning first to FIG. 2B, the reference panel sample store 256 stores a set of reference panel samples of genotype datasets for each of the $K$ labels. A reference panel for kth label is a collection of representative genetic datasets that belong to a community corresponding to kth label. For example, if the kth label represents a community of individual of an Asian reference panel, the reference panel samples in the kth-label reference panel are representative Asian genotype datasets. For more details on how reference panel samples may be identified and/or generated, U.S. Patent Application Publication 2016/0350479 published on December 1, 2016, entitled "Discovering Population Structure from Patterns of Identity-by-Descent," is mentioned herein for all purposes. The set of reference panel samples corresponding to the $k$th label (for $k \in \{1,...,K\}$) is referred to herein as $\boldsymbol{R}_k$. Each reference panel sample $R \in \boldsymbol{R}_k$ in the store 115 may be phased diploid genotype dataset of $L$ SNPs, $R=(R_1,...,R_L)$, where each $R_i$ (for $i \in \{1,...,L\}$) is an SNP that is an ordered pair of binary alleles (i.e., (0,0), (0,1), (1,0) or (1,1)). At some sites of SNPs, there may be missing data. The labels may each correspond to a different origin population (e.g., an ethnic group), in which case each reference panel sample $R$ may be a genotype data with a single origin from the kth origin population.

The possible labels may include both unadmixed labels and admixed labels. A collection of reference panel samples may be retrieved. The collection may include a plurality of unadmixed genetic datasets and a plurality of admixed synthetic genetic datasets. An admixed synthetic genetic dataset may be associated with both an ethnic origin and a geographical origin. For an admixed population, the same ethnic origin but with different geographical origins may be regarded as a different label. For labeling an admixed individual, at least some of the nodes in the inter-window HMM 400 may be labeled with a particular ethnic origin associated with an admixed population from a geographical origin. Other nodes in the inter-window HMM 400 may be labeled with another ethnic origin associated with the admixed population from the geographical origin. For example, in FIG. 4 shown, label 1 may be associated with Mexico-Native American while label 2 may be associated with Mexico-European.

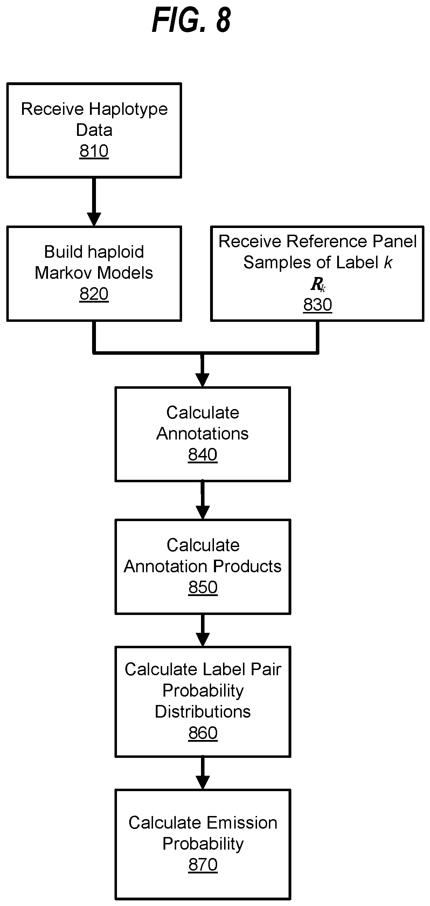

Now referring to FIG. 8, a flowchart depicting a process for calculating emission probabilities based on annotations is illustrated, according to some embodiments. The ethnicity estimation engine 245 receives 810 haplotype data of a training set. The haplotype data may be a sequence of alleles corresponding to individuals. Each sequence of haplotype data may include alleles corresponding to the $L$ SNPs of the genotypes stored in the genetic data store 264, or some subset thereof. the reference panel sample store 256 stores a set of reference samples for each of the $K$ labels. The set of reference panel samples corresponding to the $k$th label (for $k \in \{1,...,K\}$) is referred to herein as $\boldsymbol{R}_k$. Each reference panel sample $RER_k$ in the store 115 may be an unphased diploid genotype of $L$ SNPs, $R=(R_1,...,R_L)$, where each $R_i$ (for $i \in \{1,...,L\}$) is an SNP that is either an unordered pair of binary alleles (i.e., (0,0), (0,1), or (1,1)) or missing data. The labels may each correspond to a different origin (e.g., an ethnic group), in which case each reference panel sample $R$ may be a genotype from the $k$th origin population.

Some or all of the haplotype data may be phased haplotype data produced by the method described in the PCT application entitled "Haplotype Phasing Modules" (International Publication Number WO 2016/061568 A1) which was filed on October 19, 2015 and which is hereby mentioned in its entirety. In alternate embodiments, some or all of the haplotype data may be phased haplotypes produced by PHASE, BEAGLE, HAPI-UR, SHAPEIT2, IMPUTE2, or some other phase estimation method. Based on the received haplotype data, the ethnicity estimation engine 245 builds 820 haploid MMs 300 for each window w. The haploid MMs may be stored in the haploid MM Store 254.

The ethnicity estimation engine 245 also receives 830 a set of reference panel samples $\boldsymbol{R}_k$ for each label $k$ (for $1 \leq k \leq K$). The set of reference panel samples $\boldsymbol{R}_k$ may be accessed from the reference panel sample store 256. Based on the set of reference panel samples $\boldsymbol{R}_k$ for label $k$ and the haploid MMs for window w, the ethnicity estimation engine 245 calculates 840 a set of annotations $A_w(k,u)$ of every label $k$ and every state in the window w. The annotations $A_w$ may be stored in the annotation store 262. The ethnicity estimation engine 245 calculates 850 annotation products $L_w(d, p)$ based on the annotations. Based on the annotation products $L_w(d, p)$, the ethnicity estimation engine 245 calculates 860 label probability distributions. Based on the label pair probability distributions $E_{x,w}(p,q)$, the ethnicity estimation engine 245 calculates 870 the emission probability for each node. For an admixed individual, at least some of the nodes in the inter-window HMM 400 may be assigned with probabilities that are calculated based on one or more synthetic genetic datasets.

Annotation Determination

The discussion in this subsection corresponds to element 840 in FIG. 8 regarding calculation of annotation in association with the calculation of emission probabilities. The annotation $A_w(k,u)$ is based on a calculation of the conditional probability of the haploid state $u$ given the SNP sequence in the window w for the reference panel sample R that belongs to the set of reference panel samples $\boldsymbol{R}_k$ of the $k$th label. The calculation of the probability of the state $u$ given reference panel sample $R$ is based on the haploid MM 300 for window w. For a given window w, label $k$, and state $u$, the annotation $A_w(k,u)$ is equal to or positively correlated with the probability that a haplotype corresponding to label $k$ includes the haploid state $u$ in its path through window w. Equivalently, the annotation $A_w(k,u)$ may be or may represent the expected proportion of haplotypes that include haploid state $u$ in their corresponding paths for genotypes datasets selected from the set of reference panel samples $\boldsymbol{R}_k$.

In one embodiment, annotations are determined using a forward-backward algorithm. For a reference panel sample $R \in \boldsymbol{R}_k$, the forward-backward algorithm may be used to calculate a forward function $f_{R,w}$ and a backward function $b_{R,w}$. The forward function $f_{R,w}(u,v)$ may map the diploid state $(u,v)$ at level $d$ to the joint probability of the first $d$ SNPs in window w of the reference panel sample $R$ and the diploid state $(u,v)$. That is, the output of the forward function $f_{R,w}(u,v)$ is the probability, based on the haploid MM for the window w, that a genotype dataset has the first d SNPs of $R$ and that $R$ corresponds to the state $(u,v)$ at level $d$. Similarly, the backward function $b_{R,w}(u,v)$ may map the diploid state $(u,v)$ at level $d$ to the joint probability of the last $(D-d)$ SNPs in window w of the reference panel sample $R$ and the state $(u,v)$. The forward-backwards product, $f_{R,w}(u,v) \times b_{R,w}(u,v)$, may be the joint probability of all the SNPs of the reference panel sample $R$ in window w and the corresponding state $(u,v)$. In some embodiments, the outputs of the forward function $f_{R,w}$ and the backward function $b_{R,w}$ are proportional, but not necessarily equal to the probabilities of their respective diploid states.

The annotation $A_w(k,u)$ for the label $k$ and state $u$ may be given by:

$$A_w(k,u) = \frac{1}{|\boldsymbol{R}_k|} \sum_{R \in \boldsymbol{R}_k} \frac{1}{b_{R,w}(\mathbb{S}_w, \mathbb{S}_w)} \sum_{v \in \text{StatesInLevel}_w(u)} f_{R,w}(u,v) \times b_{R,w}(u,v) \qquad (1)$$

where $|R_k|$ denotes the cardinality of the set $\boldsymbol{R}_k$ (i.e., the number of reference panel samples in $\boldsymbol{R}_k$) and where StatesInLevel$_w(u)$ refers to the set of haploid states in the same level as $u$ (i.e., if $u$ is in level $d$, then StatesInLevel$_w$

$(u)$ is the set of all states at level $d$). Because ($\mathbb{S}_w, \mathbb{S}_w$) is the start state of the diploid HMM 301 for window $w$, $b_{R,w}(\mathbb{S}_w, \mathbb{S}_w)$ is equal to the likelihood of the reference panel sample $R$.

By the definition of the conditional probability, $f_{R,w}(u,v) \times b_{R,w}(u,v)/b_{R,w}(\mathbb{S}_w, \mathbb{S}_w)$ is the diploid state probability, i.e., the conditional probability that the path of a genotype dataset includes the state $(u,v)$ in the diploid HMM 301 for window $w$ given that the genotype dataset is a reference panel sample $R$. In some embodiments, the forward-backwards product $f_{R,w}(u,v) \times b_{R,w}(u,v)$ and $b_{R,w}(\mathbb{S}_w, \mathbb{S}_w)$ are calculated to be proportional, but not necessarily equivalent, to the likelihood of their respective diploid states. In such an embodiment, the diploid state probability $f_{R,w}(u,v) \times b_{R,w}(u,v)/b_{R,w}(\mathbb{S}_w, \mathbb{S}_w)$ for reference panel sample $R$ is still equivalent to the conditional probability that the path of the genotype includes the state $(u,v)$ in the diploid HMM 301 given the genotype $R$.

The summation of the diploid state $f_{R,w}(u,v) \times b_{R,w}(u,v)/b_{R,w}(\mathbb{S}_w, \mathbb{S}_w)$ over all haploid states v in level d produces the marginal probability that the first haplotype (e.g., paternal, or maternal) is in haploid state $u$ at level $d$ given the reference panel sample $R$. The diploid state probabilities for a reference panel sample $R$ may be summed over the set of diploid states that include the haploid state $u$ (i.e., diploid states $(u,v)$ and $(v,u)$ for all haploid states $v$ at the same level as the haploid state $u$) to produce a probability that the reference panel sample $R$ corresponds to the haploid state $u$. Finally, the probabilities of $u$ for each reference panel sample $R$ may be combined to produce the annotation $A_w(k,u)$. For example, $A_w(k,u)$ may be the arithmetic average of the probabilities of the haploid state $u$ for each reference panel sample $R$, therefore representing the expected proportion of reference panel samples in the set of reference panel samples $\boldsymbol{R}_k$ that include the state $u$ in their respective paths. Stated differently, the annotation $A_w(k,u)$ is the probability that the haploid state of a haplotype at a level $d$ is haploid state $u$ given that the haplotype corresponds to label $k$. In other alternatives, a different mathematical formulation other than arithmetic average may be used.

The annotations in the annotation store 262 may be calculated prior to determining labels for potentially admixed genotype datasets. In some embodiments, the annotations are updated based on labels determined for phased potentially admixed genotype datasets that are input to the system through the process described herein. In some embodiments, the annotations $A_w(k,u)$ for a label $k$ and window $w$ may be iteratively improved by determining a probability that an admixed genotype dataset corresponds to a label $k$ in window $w$ and modifying the annotations $A_w(k,u)$ accordingly.

Annotation Product Determination

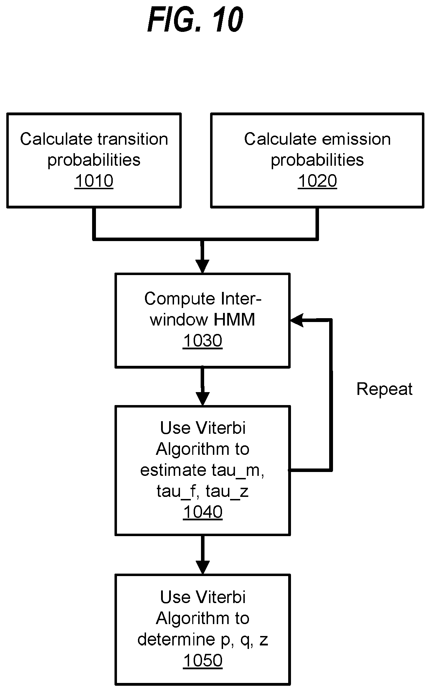

The discussion in this subsection may correspond to element 850 in FIG. 8 regarding calculation of annotation products in association with the calculation of emission probabilities. FIG. 10 is a flowchart illustrating a method for assigning labels to a genotype, according to some embodiments.

Based on the annotations $A_w(k,u)$ and the input sample genotype dataset X, which is divided into two phased haplotypes, $x_{1,w}$ and $x_{2,w}$, each a sequence of alleles $\in \{0,1\}$ corresponding to the subsequence of SNPs in window $w$, the haploid MM module 268 may calculate a label probability $E_{x,w}(p)$ for each haplotype $x \in \{x_{1,w}, x_{2,w}\}$, and each label $p \in \{1,2,...,K\}$, where $K$ is the number of possible labels. If window w is a subsequence of $D_w$ SNPs, the haploid MM module 268 determines a unique set of states $\{u_{x,w,0}, u_{x,w,1}, u_2, ..., u_{x,w,Dw}\}$ for a haplotype subsequence $x$ in window w and the label probability for label p for a haplotype x is given by

$$E_{x,w}(p) = \frac{1}{D_w} \sum_{d=0}^{D_w} \frac{A_w(p, u_{x,w,d})}{\sum_{k=1}^{K} A_w(k, u_{x,w,d})}$$

The annotation product corresponds to haplotype $x_1$ (one of the phased haplotypes) at window w. $E_{x1,w}(p)$ represents the likelihood that the window w corresponds to label $p$ given that the haplotype is $x_1$. Another annotation product $E_{x2,w}(p)$ is calculated similarly for the other phased haplotype $x_2$.

Based on the label pair probability distributions for each window w, the inter-window HMM module 272 may build an inter-window HMM 400. The transition probabilities between states in the inter-window HMM may be based on the label pair probability distribution. Also, the inter-window HMM module may use the label pair probability distribution as the probability distribution of the states in window $w$ given the SNPs in the window $w$. That is, the label pair probability distribution may be used in the inter-window HMM as the probability of the state $U_w(p,q,z)$ in window $w$ given the observation (i.e., the sequence of SNPs of the phased datasets in the window $w$). Computing the inter-window HMM 400 for the phased datasets may include determining a label probability vector and label change probabilities for the inter-window HMM.

In some embodiments, the inter-window HMM module 272 uses the label pair probability distribution to calculate the emission probabilities for states in window $w$. That is, the label pair probability may be an estimate of the probability of the sequence of SNPs in window $w$ given that the state for window $w$ is $U_w(p,q,z)$. Here $x_1$ and $x_2$ are two phased haplotypes. The emission probability is determined based on the following equation:

$$E_{x,w}(p,q,z) = \begin{cases} E_{x1,w}(p) \times E_{x2,w}(q) & \text{if } z = 0 \\ E_{x1,w}(q) \times E_{x2,w}(p) & \text{if } z = 1 \end{cases}$$

Alternatively, based on the annotations $A_w(k,u)$ and the input sample genotype dataset X, the diploid HMM module 270 may calculate a label pair probability $E_{x,w}(p,q,z)$ as an estimate of the probability of the sequence of SNPs in window w given that the state for window w is $U_w(p,q,z)$ as

$$E_{x,w}(p,q,z) = \sum_{d=0}^{D_w} \frac{L_{x,w}(p,q)}{\sum_{p',q'} L_{x,w}(p',q')}$$

where $L_{x,w}(p,q)$ is the expected annotation product given by

$$L_{x,w}(p,q) = \sum_{u,v \in \alpha_d} \frac{f_{x,w}(u,v) \times b_{x,w}(u,v)}{b_{x,w}(\mathbb{S}_w,\mathbb{S}_w)} \times \frac{A_w(p,u) \times A_w(q,v) + A_w(q,u) \times A_w(p,v)}{2}$$

And $f_{x,w}(u,v) \times b_{x,w}(u,v)/b_{x,w}(\mathbb{S}_w,\mathbb{S}_w)$ is the diploid state probability, e.g., the conditional probability that the path of a genotype dataset $x$ includes the state $(u,v)$ in the diploid HMM 301 for window w. $\mathbb{S}_w$ is the distinguished starte state in the diploid HMM 301, and $\alpha_d$ is the set of states in the diploid HMM 301 at level $d$. Note that when using the diploid HMM this way to determine the label pair probability, $E_{x,w}(p, q, z)$ does not depend on $z$.

Computing the Inter-Window HMM

FIG. 9 depicts a process for building and computing an inter-window HMM, in accordance with some embodiments. The ethnicity estimation engine 245 receives 910 an input sample genotype dataset X. The ethnicity estimation engine 245 phases 920 the input sample genotype dataset X to generate a pair of phased haplotype datasets. The pair of phased haplotype datasets may be generated using the diploid HMM 301. The ethnicity estimation engine 245 builds 930 an inter-window HMM with ($p, q, z$) as labels of the hidden states. The ethnicity estimation engine 245 initializes 940 the label change probabilities $\tau^m$ and $\tau^f$ and the label switch probability $\tau^z$ when computing the inter-window HMM. The label change probabilities and the label switch probability may be initialized to a low value (e.g., between 0.5 to $10^{-4}$) and iteratively updated. After a predetermined number of rounds of iteration and/or after the values of label change probabilities and label switch probability have converged, the ethnicity estimation engine 245 calculates 950 the transition probabilities for different possible transitions.

The calculation of label probability vector, label change probabilities, and label switch probability may be carried through one or more forward-backward algorithms. Computing the inter-window HMM may include calculating a label probability vector and the label change probabilities. The label probability vector may be initialized to a uniform distribution. The label probability vector may be iteratively updated with expectation-maximization (e.g., with the Baum-Welch algorithm). In some embodiments, the inter-window HMM module may perform $N$ iterations of the Baum-Welch algorithm to calculate the label probability vector. The label change probability and the label switch probability may be initialized to a low value (e.g., between 0.5 to $10^{-4}$) and iteratively updated.

In one example, a label pair expectation $\mathbf{E}[\pi_{X,(p,q)}]$ is calculated for each of the pair of labels ($p,q$). The label pair expectation $\mathbf{E}[\pi_{X,(p,q)}]$ is the sum of the probabilities of each state $U_{w,(p,q)}$ for each window $w$ and is therefore equal to the expected number of windows w that have a hidden state $U_{w,(p,q)}$ corresponding to the label pair ($p,q$). Each label probability $\pi_{(p,q)}$ is updated to a new value: the label pair expectation $\mathbf{E}[\pi_{X,(p,q)}]$ of the label pair ($p,q$) divided by the sum of label pair expectations for all label pair probabilities, so that the label probabilities $\pi_{(p,q)}$ sum to unity (i.e., $\Sigma\pi = 1$).

In one example, the label change probability $\tau^m$ and $\tau^f$ are each initialized to $10^{-3}$ and then iteratively updated to the expected number of transitions that change label assignments. Put differently, the label change probability is updated to the complement of the expected number of transitions between states that correspond to the same labels (e.g., one minus probability of no change in label) divided by the expected number of all transitions between states. Likewise, the label switch probability $\tau^z$ may also be initialized to a low value then iteratively updated.

Label Assignment

FIG. 10 illustrates a process of providing a label assignment of an input genotype dataset, in accordance with an embodiment. Using a set of training samples such as those obtained from different reference panels, the ethnicity estimation engine 245 calculates 1010 transition probabilities for different possible transitions for an inter-window HMM in the training of the inter-window HMM. The calculation of transition probabilities may correspond to the process shown in FIG. 10. Based on the reference panel samples and the input sample genotype dataset X, the ethnicity estimation engine 245 calculates 1020 the emission probabilities for different hidden states in the inter-window HMM. The calculation of the emission probabilities may correspond to the process shown in FIGS. 3A-5. The ethnicity estimation engine 245 updates and builds (e.g., computes) 1030 an inter-window HMM using the pair of phased haplotype datasets derived from the input sample genotype dataset X. The computation may include generating data representing a directed acyclic graph that may include the structure of the inter-window HMM 400. The ethnicity estimation engine 245 uses 1040 Viterbi algorithm to estimate the label change probabilities and the label switch probability in the updated inter-window HMM. Based on the Viterbi path, the labels corresponding to the input sample genotype dataset X are determined. The determined Viterbi path may be used as one of the samples of a new set of training samples (which include the selected training samples from reference panels and the determined Viterbi path as an additional sample) to update and re-build 1030 the inter-window HMM. The process of 1030 and 1040 can be repeated for a predetermined number of iterations (e.g., 10 times) and/or repeated until the label changes probabilities and the label switch probability converge. The ethnicity estimation engine 245 uses 1050 the Viterbi algorithm one more time to determine the Viterbi path corresponding to the input sample genotype dataset X to assign the value of labels $p$, $q$ and $z$ in each window. A final path may be determined after repeating the Viterbi algorithm multiple times. The final path may traverse the directed acyclic path and may represent the a statistically likely path among other possible paths in traversing the directed acyclic graph.

In some embodiments, the label assignment may involve determining a proportion of the input sample genotype dataset X that corresponds to each label. For example, the label assignment module 276 of the ethnicity estimation engine 245 may determine that 25% of the input sample genotype dataset X corresponds to label 1, 0% corresponds to label 2, 50% corresponds to label 3, and 25% corresponds to label 4. The proportion of each label may be based on the states in the Viterbi path, based on the probability of being in each state (e.g., as calculated with the forward-backward algorithm), or otherwise based on the inter-window HMM. The determination of these proportions may also be based on a weight assigned to each window w. The weight of each window w may be based on the size of the window (e.g., in the number of bases). The weighting of each window w may be adjusted based on portions of the windows w that overlaps with other windows.

In some embodiments, the label assignment module 276 assigns a pair of ordered classification labels to each window w of the input sample genotype dataset X. In some embodiments, the label assignment module 276 determines the Viterbi path through the inter-window HMM 400. In alternate embodiments, the label assignment module 276 computes a number (e.g., 1000) of stochastic paths through the inter-window HMM and determines a range of each label's proportion based on the states taken by the stochastic process. For example, the label assignment module 276 may determine that 18-30% of the input sample genotype dataset X corresponds to a particular label. The range may be based on the maximum and minimum proportion of the genotype dataset X that corresponds to a label in the stochastic paths. Alternately, the range may be based on percentiles of the proportions of the input sample genotype dataset X that corresponds to a label in the stochastic paths. For example, the upper bound of a range for label k may be based on a 95th percentile of the proportions of the states that correspond to label k in the stochastic paths and the lower bound may be based on the 5th percentile. The most probable path or one of 95th percentile (or another suitable percentile) likely stochastic paths among other possible paths in traversing the directed acyclic graph may be referred to as a statistically likely path. Further details regarding determining different paths and range are discussed with reference to the Section below entitled "Range Determination."

In some embodiments, the label assignment module 276 assigns labels to specific portions of the input sample genotype dataset X. The label assignment module 276 may specifically assign labels to a portion of the input sample genotype dataset X that corresponds to one or more overlapping regions with a second genotype. For example, if the input sample genotype and the second genotype dataset are the genotypes of related individuals (e.g., first cousins), then the one or more overlapping regions are the regions of genetic information that correspond to one or more shared ancestors (e.g., a grandmother and a grandfather shared by the cousins). If, in an overlapping region, there is only one haplotype (in each genotype) that overlaps between the input sample genotype dataset X and the second genotype dataset, the label assignment module 276 may assign labels specifically to the overlapping haplotype.

For an admixed individual, the label determination and assignment may be similar, but each label may include an ethnic origin and a geographical region. For example, a label for a particular window may be labeled with the ethnic origin Native America and with the geographical region of Mexico. A genetic segment that includes one or more consecutive windows may be assigned with the same label having the same ethnic-origin-geographical-region pair. The genetic segment may be added to one of the synthetic genetic datasets as part of a reference panel sample for an admixed population.

Providing Information on Ethnic Origin

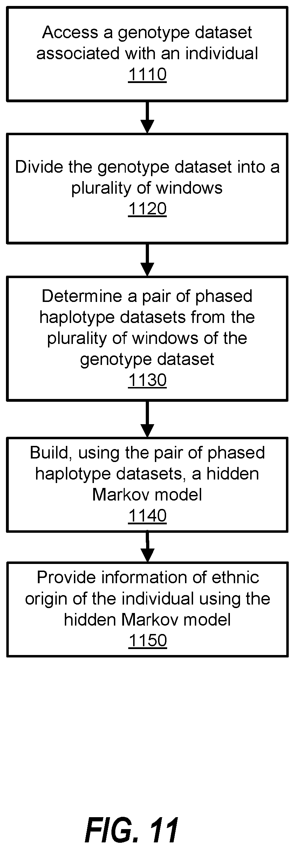

FIG. 11 depicts a process of providing information on ethnic origin for an individual such as an end user, in accordance with an embodiment. The ethnicity estimation engine 245 accesses 1110 a genotype dataset associated with an individual. The genotype dataset may be stored in a data store after the biological sample (such as blood or saliva sample) of the individual to is analyzed to generate the genotype dataset sample. The ethnicity estimation engine 245 divides 1120 the genotype dataset into a plurality of windows. Each window comprises a plurality of SNPs. The ethnicity estimation engine 245 determines 1130 a pair of phased haplotype datasets from the plurality of windows of the genotype dataset. The ethnicity estimation engine 245 builds 1140 a hidden Markov model using the pair of phased haplotype datasets. The HMM may be an inter-window HMM. Using the HMM computed and trained, the ethnicity estimation engine 245 assign labels to each window correspond to the genotype dataset based on the Viterbi path of the HMM. The nodes traversed by the Viterbi path each is associated with a first parent label and a second parent label. The statistic of the plurality of labels of the nodes can be determined. For example, the distribution of each label in terms of percentage may be determined. The ethnicity estimation engine 245 then provides 1150 information of the ethnic origin of the individual using the results of the label assignment of the HMM. The ethnicity estimation engine 245 may provide a front-end user graphical interface for the presentation and display of the information of the ethnic origin of the individual, who may be an end user of the ethnicity estimation engine 245.

The information on the ethnic origin of the individual may take different forms. In a first example, the information may simply be the most likely ethnical origin of the individual. The ethnicity estimation engine 245 may simply inform the individual that he/she is of a certain origin. In a second example, the information may include paternal origin and maternal origin. In a third example, the information may include the statistic and/or the detailed proportions of genetic origins. For instance, the ethnicity estimation engine 245 may inform the individual that, on one parental side, 80% of the genes of the individual are inherited from European ancestors (e.g., based on 80% of first parent labels being European), while 15% of the genes are inherited from Asian ancestors, etc. In a fourth example, the information may take the form of a visualization of the individual's ancestry composition by chromosome painting. For example, an end user may want to know what parts of his DNA come from his African ancestors. The ethnicity estimation engine 245 may highlight portions of the chromosomes that correspond to windows that are labeled as African. In a fifth example, the information may take the form that is specifically related to a trait or phenotype. For example, an end user may select a question regarding from whom ancestor she received her blue eyes. The ethnicity estimation engine 245 may provide an answer to this type of questions such as in the form of "You inherit X trait from Y ancestor." In a sixth example, the information may take the form that is related to an ancestor. For instance, the ethnicity estimation engine 245 may provide that the end user share 60% of DNA when compared to a particular ancestor. In a seventh example, the information may take the form that compares the similarity and differences of the genotypes between the individual's father and mother (or paternal ancestors and maternal ancestors). In an eighth example, the information may take the form that focuses on other people who are genetically related to the individual. For instance, the ethnicity estimation engine 245 may provide the percentage of people of a certain ethnicity at trait loci has blue eyes. Other forms of information of ethnic origin of the individual are also possible.

For a target admixed individual, the information of ethnic origins of the individual may include information of genetic composition of the individual having a particular ethnic origin. For example, after a statistically likely path is determined based on the result of the HMM, the number of nodes that are labeled with a particular ethnic origin and a particular geographical region and that are included in the path may be calculated and compared to the total numbers of windows to determine the percentage of the particular ethnic origin of the target admixed individual. In one embodiment, in providing the genetic composition of a particular ethnic origin of an admixed individual, the ethnicity estimation engine 245 may distinguish the particular ethnic origin from two different geographical regions. For example, the ethnicity estimation engine 245 may report that the target admixed individual has 20% Native American genetic segments from Mexican ancestors and 15% Native American genetic segments from Brazilian ancestors. In another embodiment, the ethnicity estimation engine 245 may distinguish the geographical regions when assigning labels but combine the same ethnic origin from different geographical origins together when reporting the result. For example, the ethnicity estimation engine 245 may report that the target admixed individual has in total 35% Native American origin, regardless of whether the genetic segments are labeled with Mexico or Brazil.

Admixed Reference Panel Generation

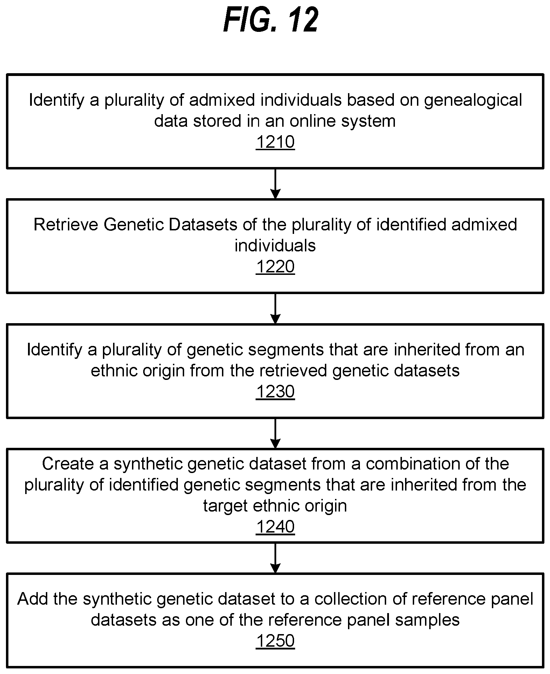

FIG. 12 is a flowchart depicting an example process of generating an admixed reference panel sample, which may be a synthetic genetic dataset, in accordance with an embodiment. An online system, such as the ethnicity estimation engine 245, may identify 1210 a plurality of admixed individuals. The identification of admixed individuals may be based on genealogical data stored in the online system. As indicated by the genealogical data, each identified admixed individual may commonly have at least one ancestor originated from a target geographical region. For example, the target geographical region may be Mexico. The identified admixed individual may each have at least one ancestor from Mexico. The genealogical data may be any suitable identification information that indicates the born location or nationality of the ancestors. For example, the genealogical data may include a pedigree of one of the identified admixed individuals with geographical location input by the individual. The genealogical data may also be census data or birth data of the ancestors. Genealogical data may include data from one or more of a pedigree of an individual, the Ancestry World Tree system, a Social Security Death Index database, the World Family Tree system, a birth certificate database, a death certificate database, a marriage certificate database, an adoption database, a draft registration database, a veterans database, a military database, a property records database, a census database, a voter registration database, a phone database, an address database, a newspaper database, an immigration database, a family history records database, a local history records database, a business registration database, a motor vehicle database, and the like.

The online system may retrieve 1220 genetic datasets of the plurality of identified admixed individuals. The genetic datasets may be a genotype dataset or a haplotype dataset. The online system may also phase a genotype dataset into a pair of haplotype datasets. For an admixed individual who is identified, the genetic dataset may include a plurality of SNP sites of the admixed individual. The online system may divide the genetic dataset into a plurality of windows. Each window may correspond to a genetic locus or may include one or more SNP sites.

The online system may identify 1230 a plurality of genetic segments that are inherited from an ethnic origin from the retrieved genetic datasets. The identification of the plurality of genetic segments may include one or more sub-steps. For example, for each retrieved genetic dataset, the online system may input the genetic dataset into an HMM, such as the inter-window HMM 400, to generate labels for each of the window in the genetic dataset. While not all identified admixed individuals may have any genetic segment that is inherited from an ethnic origin, a subset of the identified admixed individuals may include some of the windows that are labeled with the ethnic origin. The online system may identify one or more genetic segments that are inherited from the ethnic origin based on the labels. For example, there might be a set of consecutive windows that are labeled with a target ethnic origin and the target geographical region. The online system may combine those windows and treated it as a genetic segment. A genetic segment may include a single window or a plurality of windows.

In one embodiment, the identification of genetic segments that belong to a target ethnic origin may include the use of the process that is described further detail in FIG. 3A through FIG. 11, including the use of one or more HMMs and CNNs. For example, for each genetic dataset, the online system may generate data representing a directed acyclic graph that include a plurality of node groups. The directed acyclic graph may represent a trellis of an HMM. Each node group of the graph may represent a window that corresponds to a genetic segment of the identified admixed individual. Each of most of the windows (e.g., except windows representing inter-chromosome states) may be represented by a plurality of nodes. Each node may include a pair of labels that corresponds to the pair of haplotypes. Each label may represent one of the possible ethnic origins. The possible ethnic origins including the target ethnic origin and other ethnic origins. The online system may determine a path traversing the directed acyclic graph. The path may represent a statistically likely path among other possible paths in traversing the directed acyclic graph. For example, a statistically likely path may be the most probable path or a path that is more probable than 95% (or another suitable threshold) than other possible paths. A statistically likely path may also be an average of a selection of multiple probable paths. The online system may identify one or more nodes included in the path that has at least a label of the target ethnic origin. The genetic segments that correspond to the identified nodes may be identified as the genetic segments that are inherited from the target ethnic origin.

The online system may identify genetic segments inherited from the target ethnic origin from different admixed individuals. The genetic dataset of each admixed individual may contribute to a different genetic segment. For example, at least a first genetic segment identified from a first admixed individual and a second genetic segment identified from a second admixed individual may be among the identified genetic segments that are inherited from the target ethnic origin. The first and second genetic segments are different segments and may be located at different genetic loci.

The online system may create 1240 a synthetic genetic dataset from a combination of the plurality of identified genetic segments that are inherited from the target ethnic origin. The synthetic genetic dataset may be divided into a plurality of windows. Each window may be associated with a genetic sequence that is determined from the genetic data of a different admixed individual. The synthetic genetic dataset may serve as a representative of the target ethnic origin in the target geographical region. For example, the synthetic genetic data may serve as a reference panel sample for the ethnic origin Native American for the admixed population in Mexico. The online system may create a second synthetic genetic dataset that includes a second combination of genetic segments that are inherited from the same target ethnic origin (e.g.,

Native American), but for a second geographical region (e.g., Brazil) different from the target geographical region.

After the synthetic genetic dataset is created, the online system may add 1250 the synthetic genetic dataset to a collection of reference panel datasets as one of the reference panel samples. The collection of reference panel datasets may be used to provide possible ethnic origin labels to other genetic datasets of other individuals. The collection of reference panel datasets may include the synthetic genetic dataset and genetic datasets of unadmixed individuals. The collection of reference panel datasets may be used for the annotation process in connection with the inter-window HMM 400.

Determination of Ancestors of Admixed Individuals

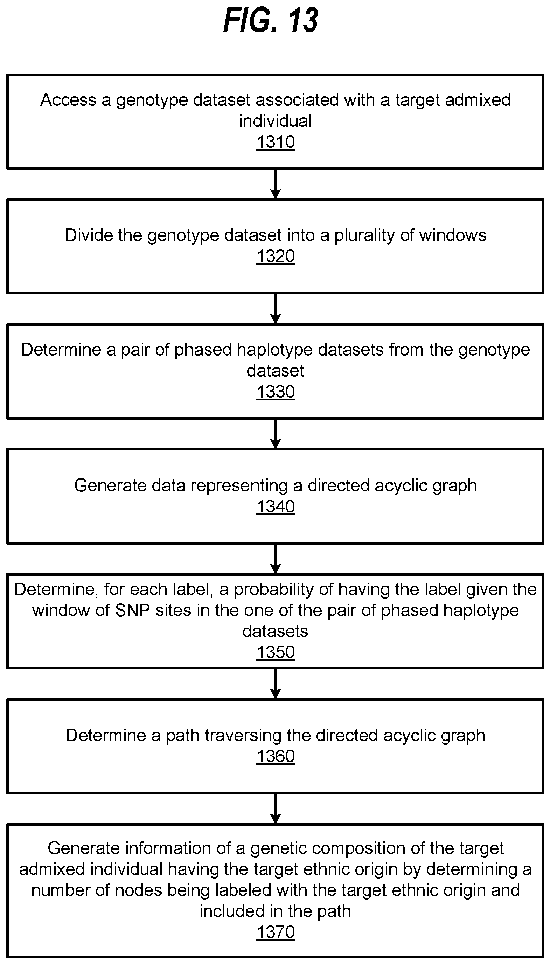

FIG. 13 is a flowchart depicting an example process of determining ethnic origin composition of an admixed individual, in accordance with an embodiment. The process may be similar to the process of using an inter-window HMM 400 as discussed in FIG. 3A through FIG. 11. An online system may access 1310 a genotype dataset associated with a target admixed individual. The online system may divide 1320 the genotype dataset into a plurality of windows. Each window may include a set of SNP sites. The online system may determine 1330 a pair of phased haplotype datasets from the genotype dataset. Each phased haplotype dataset may also be divided into the plurality of windows. The online system may retrieve a collection of reference panel datasets. The collection of reference panel datasets may include one or more synthetic genetic datasets, each of which corresponds to a target ethnic origin association with an admixed population from a geographical region.

The online system may generate 1340 data representing a directed acyclic graph. For example, the directed acyclic graph may represent a trellis of an inter-window HMM 400. The graph may include a plurality of node groups and a plurality of nodes. Each node may include a pair of labels representing a pair of possible ethnic origins for a first parent and a second parent. Each node may additionally include a switch label representing a switch of the order of the first parent label and the second parent label. Various labels may include different ethnic origins of the same admixed population from the same geographical region. For example, for an admixed Hispanic individual from Mexico, various labels may include Native American, European, African, etc.

The online system may determine 1350, for each label, a probability of having the label given the window of SNP sites in the one of the pair of phased haplotype datasets. The determination may be based on comparing the windows of SNP sites to the collection of reference panel datasets. The probability of labels associated with admixed ethnic origin may be assigned based on the one or more synthetic genetic datasets. The online system may determine 1360 a path traversing the directed acyclic graph. The path may represent a statistically likely path among other possible paths in traversing the directed acyclic graph. The online system may generate 1370 information of genetic composition of the target admixed individual having the target ethnic origin by determining the number of nodes being labeled with the target ethnic origin and included in the path. The genetic segments that are identified as being inherited from the target ethnic origin may be fed back to the synthetic genetic datasets to reinforce or improve the synthetic genetic datasets.

Range Determination

In an embodiment, the labeling model 290 outputs for each sample, a maximum likelihood estimate based on a reference path, such as the Viterbi path, of a directed acyclic graph such as the inter-window Hidden Markov Model (HMM) 400, together with a set of sampled estimates (e.g., 1,000 sampled estimates), each derived from a sampled path sampled from the HMM randomly or at least partially randomly based on certain criteria such as transition probabilities. The range module 280 analyzes these sampled estimates subsequently to estimate a range surrounding a reference estimate derived from the reference path. In some cases, the reference estimate may also be referred to as a Viterbi estimate. In one embodiment, the range module 280 uses the lowest and highest sampled estimates to define the lower and upper bound of the reference estimate. Experiments suggest that such approach tends to underestimate the uncertainty associated with the reference estimate. In another embodiment, the range module 280 estimates an interval surrounding the reference estimate. The range module 280 maximizes the probability that the reported range contains the true ancestry proportion (recall), while also maintaining a reasonably narrow range size.

A reference path may be any statistically likely path that traverses the directed acyclic graph (e.g., a path that is statically more likely than 95% of all possible paths traversing the directed acyclic graph). In one embodiment, the reference path is the single most likely path among all possible paths, which may also be referred to as the Viterbi path. For a directed acyclic graph, such as the trellis representing the inter-window HMM 400, the probability of any given path is determined based on the transition probability and the emission probability associated with the nodes that are traversed by the path. For example, referring to FIG. 4, a path that traverses the start state 410, the node 404, the node 406, will have an overall probability value equal to the emission probability of the node 404 multiplied by the transition probability of a transition from node 404 to node 406 multiplied by the emission probability of the node 406. As the path further extends to window 3, additional terms of transition probability and emission probability will be multiplied to the overall probability value of the path. The mostly likely path has the highest overall probability value among all possible paths. A statistically likely path has overall probability that is higher than a predetermined percentage (e.g., 95%) of all possible paths. In determining the mostly likely path, a Viterbi algorithm may be used to prune unlikely paths to reduce the amount of calculation.

In one embodiment, a path may also be sampled from the directed acyclic graph. A sampled path may also be referred to as a stochastic path. The sampling of paths may be performed based on different approaches in various embodiments. For example, A predetermined percentage threshold may be defined. Paths that have a probability value that is higher than the predetermined percentage threshold of all possible paths may be randomly selected as sampled paths. In one embodiment, let *CHOOSE* be an operator that chooses an argument with a probability relative to an expression so that $\underset{x \in D}{CHOOSE} f(x)$ returns *x* with probability $\frac{f(x)}{\sum_{x' \in D} f(x')}$. The domain for *p, q* in is all ordered pairs of populations (i.e., all *p, q* such that $1 <= p <= K$ and $1 <= q <= K$ for *K* populations). Then a stochastic path Q for a genetic sequence x is defined over a set of windows $1 \le w \le W$ as follows. For windows that are last in a chromosome, *c,*

$$Q_{x,C(c+1)-1} = \underset{p,\,q}{CHOOSE} F_x(S_{C(c+1)-1,p,q})$$

For other windows w,

$$Q_{x,w} = \underset{p,\,q}{CHOOSE} F_x(S_{w,p,q}) \times P_x(S_{w,p,q} \to S_{w+1,Q_{x,w+1}}) \times E_{x,w+1}(Q_{x,w+1})$$

In these equations, $F_x(S)$ denotes the forward probability, i.e., the sum of probability of all paths through the HMM that start in the start state and end in state *S* (including the emission of state *S*); $S_{w,p,q}$ denotes the state of window w, where *p,q* is the two ethnicity assignment at window *w*; $S_{C(c+1)-1,p,q}$ denotes the last state in a chromosome c; $P_x(S)$ denotes the transition probability, and $E_{x,w+1}(S)$ is the emission probability at window *w + 1.*

The range module 280 uses the mean and standard deviation of the set of sampled estimates (e.g., 1,000 sampled estimates) to calculate an interval surrounding the reference estimate. The approach also accounts for the reference estimate, and the population for which range is calculated. Accordingly, the range module 280 obtains the lower and upper bounds of the interval by linearly scaling the standard deviation of the sampled estimates (e.g., 1,000 sampled estimates) with factors that are specific to both population and Viterbi value (lower and upper bound factors can be different to reflect upward or downward bias of the estimate).

The range module 280 takes the set of sampled estimates, referred to as P, and computes the standard deviation, S, and mean, M, of P using equation (1).

$$S = sd(P); \; M = mean(P) \tag{1}$$

The range module 280 also identifies the scaling factors $\lambda_1$ and $\lambda_2$ for the upper and lower bounds of the range respectively. The range module 280 determines the reported confidence interval as $[M - S * \lambda_1, M + S * \lambda_2]$, where, $\lambda_1$ and $\lambda_2$ are specific for population, and to the bin that the reference estimate falls into. In the event that the Viterbi estimate falls outside of this confidence interval, the interval may be adjusted to include the Viterbi estimate as the upper or lower bound.

The values for λ1 and λ2 are determined using a set of simulated individuals with known ethnicity proportions. Training for the values of λ1 and λ2 is done by performing an exhaustive joint search over a 2-dimensional grid in 0.5 increments of λ1 and λ2 and finding the combination of values that maximizes the following statistic F as determined by equation (2).

$$F = recall - K * (S * \lambda_1 + S * \lambda_2) \tag{2}$$

In equation (2), *K* is a value that adjusts the importance placed on reporting a narrow range (higher *K* increases the emphasis on narrower range). Different values of *K* can be used to adjust the trade between higher recall and narrower range size. Performance of this approach may be evaluated by measuring recall with the optimized lambda values on an independent set of simulated admixed individuals

In one embodiment, the ethnicity estimation engine 245 may transmit the determination results to the end user for display at a graphical user interface. The percentage reported to a user may be the most likely percentage within a range of percentages. For example, the ethnicity estimation engine 245 might report an end user as 40% England and Wales with a confidence range of 30-60%. This can be interpreted by the end user that he/she is most likely 40% England and Wales but that he/she could be anywhere between 30 and 60% England and Wales.

The ethnicity estimation engine 245 runs a reference estimate on a user's DNA sample (e.g., a genome wide estimate) and reports that back as the user's most likely ethnicity estimate. The range is based on a set of randomly sampled paths (e.g., 1000 sampled paths). For example, if a window has an 80% chance of being from England and Wales, then it has a 20% chance of being from some other region. The confidence interval captures these sorts of lower chances across a user's DNA.

The ethnicity estimation engine 245 uses a set of sampled estimates, to estimate the confidence interval surrounding the reference estimate that is reported to the user. The system maximizes the probability that the reported range contains the true ancestry proportion (recall), while also maximizing precision by maintaining a fairly narrow range.

The ethnicity estimation engine 245 takes the mean and standard deviation of the 1000 sampled estimates and uses this to calculate a confidence range surrounding the reference estimate. When calculating this range, the ethnicity estimation engine 245 takes into account the value of the Viterbi estimate, and the population for which the range is calculated.

In an embodiment, the process is tested using the same synthetic admixed individuals used for the cross validation studies to determine how often it correctly gets the known ethnicity percentage within the range. In other words, how often does the range overlap the known ethnicity. The process performs very well for some populations and less well for others. Since the true ethnicity is known, the system incorporates correction factors specific for each population to maximize the probability that the true ethnicity falls within the confidence level.

Confidence Module

Confidence, in this context, may be referred to the likelihood an individual truly inherits DNA from ancestor(s) of a certain population. In an embodiment, the confidence module 282 implements a machine learning approach, such as a random forest approach, to assign a confidence level, categorized as a set of predetermined levels (e.g., low, medium or high) for each estimated population.

In one embodiment, the machine learning model may be a random forest model. The random forest model comprises a list of binary classifiers, with each classifier taking a subset of input features and voting between binary values, for example, 1 indicating "yes, this individual inherits DNA from this population" and 0 indicating "no, this individual does not inherit DNA from this population". Features used by these classifiers include quantiles calculated from the set of sampled estimates and the reference estimate passing from the labeling model 290.

Feature vector F is defined by equation (3).

$$F = [\text{Viterbi estimate}, 5\%, 10\%, 15\%, \ldots, 95\% \text{ quantile of the set of samples estimates}]$$

$$(3)$$

The binary classifiers are trained using a set of simulated individuals with known ancestry proportions. Training for classifiers is done using a random forest algorithm. In an embodiment, a random forest model is trained separately for each population.

For each estimated population, the percentage of binary classifications supporting ancestry from that population is convert into a score between 0 and 1. A set of simulated data with known ancestry proportions is used as validation set to calibrate between Random Forest score and the confidence. As an example, random forest score thresholds for assigning low, medium, high confidence are selected using the following rules in table I below.

Table I. Categories of confidence classification and the corresponding validation set confidence

| Confidence level | Random Forest score threshold |
|---|---|
| High | 95% confidence on the validation set |
| Medium | 60-95% confidence on the validation set |
| Low | Below 60% confidence on the validation set |

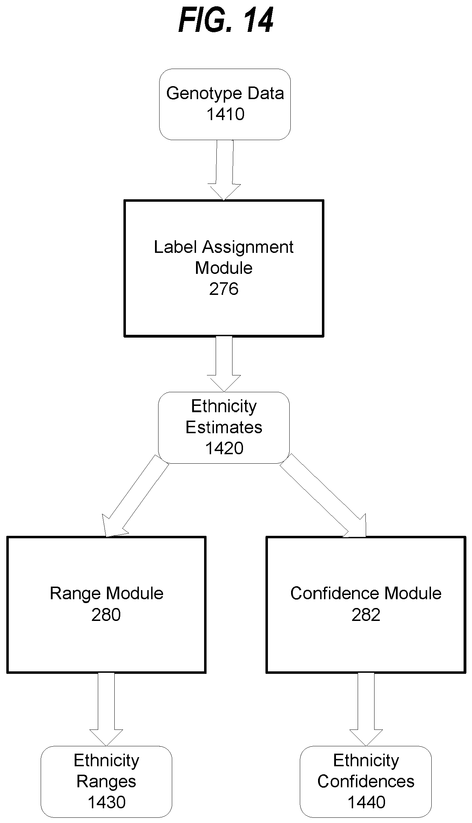

FIG. 14 shows the overall process of generating ethnicity ranges and ethnicity confidences, in accordance with an embodiment. As shown in FIG. 14, the labeling model 290 receives genotype data 1410 as input and generates ethnicity estimates 1420. The range module 280 receives the ethnicity estimates 1420 generated by the labeling model 290 and generates ethnicity ranges 1430 based on the ethnicity estimates 1420. The confidence module 282 receives the ethnicity estimates 1420 generated by the labeling model 290 and generates ethnicity confidences 1440 based on the ethnicity estimates 1420.

COMPUTING MACHINE ARCHITECTURE

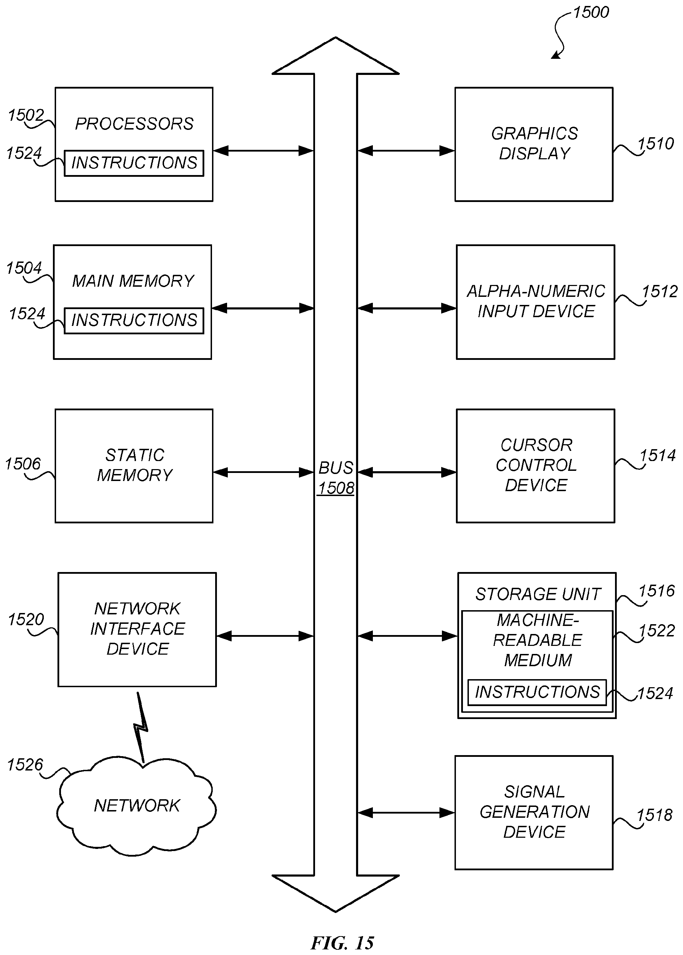

FIG. 15 is a block diagram illustrating components of an example computing machine that is capable of reading instructions from a computer-readable medium and execute them in a processor (or controller). A computer described herein may include a single computing machine shown in FIG. 15, a virtual machine, a distributed computing system that includes multiples nodes of computing machines shown in FIG. 15, or any other suitable arrangement of computing devices.

By way of example, FIG. 15 shows a diagrammatic representation of a computing machine in the example form of a computer system 1500 within which instructions 1524 (*e.g.*, software, program code, or machine code), which may be stored in a computer-readable medium for causing the machine to perform any one or more of the processes discussed herein may be executed. In some embodiments, the computing machine operates as a standalone device or may be connected (e.g., networked) to other machines. In a networked deployment, the machine may operate in the capacity of a server machine or a client machine in a server-client network environment, or as a peer machine in a peer-to-peer (or distributed) network environment.

The structure of a computing machine described in FIG. 15 may correspond to any software, hardware, or combined components shown in FIGS. 1 - 2B, including but not limited to, the ethnicity estimation engine 245, various computing devices engines, interfaces, terminals, and machines. While FIG. 15 shows various hardware and software elements, each of the components described herein may include additional or fewer elements.

By way of example, a computing machine may be a personal computer (PC), a tablet PC, a set-top box (STB), a personal digital assistant (PDA), a cellular telephone, a smartphone, a web appliance, a network router, an internet of things (IoT) device, a switch or bridge, or any machine capable of executing instructions 1524 that specify actions to be taken by that machine. Further, while only a single machine is illustrated, the term "machine" and "computer" may also be taken to include any collection of machines that individually or jointly execute instructions 1524 to perform any one or more of the methodologies discussed herein.

The example computer system 1500 includes one or more processors 1502 such as a CPU (central processing unit), a GPU (graphics processing unit), a TPU (tensor processing unit), a DSP (digital signal processor), a system on a chip (SOC), a controller, a state equipment, an application-specific integrated circuit (ASIC), a field-programmable gate array (FPGA), or any combination of these. Parts of the computing system 1500 may also include a memory 1504 that store computer code including instructions 1524 that may cause the processors 1502 to perform certain actions when the instructions are executed, directly or indirectly by the processors 1502. Instructions can be any directions, commands, or orders that may be stored in different forms, such as equipment-readable instructions, programming instructions including source code, and other communication signals and orders. Instructions may be used in a general sense and are not limited to machine-readable codes.

One and more methods described herein improve the operation speed of the processors 1502 and reduces the space required for the memory 1504. For example, the machine learning methods described herein reduces the complexity of the computation of the processors 1502 by applying one or more novel techniques that simplify the steps in training, reaching convergence, and generating results of the processors 1502. The algorithms described herein also reduces the size of the models and datasets to reduce the storage space requirement for memory 1504.

The performance of certain of the operations may be distributed among the more than processors, not only residing within a single machine, but deployed across a number of machines. In some example embodiments, the one or more processors or processor-implemented modules may be located in a single geographic location (*e.g.*, within a home environment, an office environment, or a server farm). In other example embodiments, the one or more processors or processor-implemented modules may be distributed across a number of geographic locations. Even though in the specification or the claims may refer some processes to be performed by a processor, this should be construed to include a joint operation of multiple distributed processors.

The computer system 1500 may include a main memory 1504, and a static memory 1506, which are configured to communicate with each other via a bus 1508. The computer system 1500 may further include a graphics display unit 1510 (*e.g.,* a plasma display panel (PDP), a liquid crystal display (LCD), a projector, or a cathode ray tube (CRT)). The graphics display unit 1510, controlled by the processors 1502, displays a graphical user interface (GUI) to display one or more results and data generated by the processes described herein. The computer system 1500 may also include alphanumeric input device 1512 (*e.g.,* a keyboard), a cursor control device 1514 (*e.g.,* a mouse, a trackball, a joystick, a motion sensor, or other pointing instrument), a storage unit 1516 (a hard drive, a solid state drive, a hybrid drive, a memory disk, etc.), a signal generation device 1518 (*e.g.,* a speaker), and a network interface device 1520, which also are configured to communicate via the bus 1508.

The storage unit 1516 includes a computer-readable medium 1522 on which is stored instructions 1524 embodying any one or more of the methodologies or functions described herein. The instructions 1524 may also reside, completely or at least partially, within the main memory 1504 or within the processor 1502 (*e.g.*, within a processor's cache memory) during execution thereof by the computer system 1500, the main memory 1504 and the processor 1502 also constituting computer-readable media. The instructions 1524 may be transmitted or received over a network 1526 via the network interface device 1520.

While computer-readable medium 1522 is shown in an example embodiment to be a single medium, the term "computer-readable medium" should be taken to include a single medium or multiple media (*e.g.,* a centralized or distributed database, or associated caches and servers) able to store instructions (*e.g.*, instructions 1524). The computer-readable medium may include any medium that is capable of storing instructions (*e.g.*, instructions 1524) for execution by the processors (e.g., processors 1502) and that cause the processors to perform any one or more of the methodologies disclosed herein. The computer-readable medium may include, but not be limited to, data repositories in the form of solid-state memories, optical media, and magnetic media. The computer-readable medium does not include a transitory medium such as a propagating signal or a carrier wave.

Additional Considerations

The embodiments described herein create reference samples from extracted segments of ethnicity from admixed samples. This can be used to enhance admixed reference panel. The embodiments described herein can also be used to further break down ethnicity regions to identify potential new sub-regions, or refined ancestry estimation. For example, new regions can be made for certain admixed reference panel. The embodiments described herein can also perform admixture mapping to discover ethnicities that correlate with traits in admixed individuals and to study the time of admixture events. For example, the length of ethnicity segments can provide insight on when the admixture happened.

The ethnicity estimation engine 245 comprises one or more processors and one or more non-transitory computer readable storage mediums. The one or more processors may implement the functions attributed above to modules. The modules may be hardware modules (i.e., computer hardware specially configured to perform specific functions), software modules, or some combination thereof. The non-transitory computer readable mediums may store computer instructions that, when executed, perform the methods described herein. In some embodiments, the ethnicity estimation engine 245 is a single computing system. In alternate embodiments, the ethnicity estimation engine 245 may be a distributed system including spatially-separated databases and computing systems (e.g., servers) that communicate via a network.

The ethnicity estimation engine 245 is implemented using one or more computers having one or more processors executing application code to perform the steps described herein, and data may be stored on any conventional non-transitory storage medium and, where appropriate, include a conventional database server implementation. For purposes of clarity and because they are well known to those of skill in the art, various components of a computer system, for example, processors, memory, input devices, network devices and the like are not shown in FIG. 1. In some embodiments, a distributed computing architecture is used to implement the described features. One example of such a distributed computing platform is the Apache Hadoop project available from the Apache Software Foundation.

In addition to the embodiments specifically described above, those of skill in the art will appreciate that the invention may additionally be practiced in other embodiments. Within this written description, the particular naming of the components, capitalization of terms, the attributes, data structures, or any other programming or structural aspect is not mandatory or significant unless otherwise noted, and the mechanisms that implement the described invention or its features may have different names, formats, or protocols. Further, the system may be implemented via a combination of hardware and software, as described, or entirely in hardware elements. Also, the particular division of functionality between the various system components described here is not mandatory; functions performed by a single module or system component may instead be performed by multiple components, and functions performed by multiple components may instead be performed by a single component. Likewise, the order in which method steps are performed is not mandatory unless otherwise noted or logically required. It should be noted that the process steps and instructions of the present invention could be embodied in software, firmware or hardware, and when embodied in software, could be downloaded to reside on and be operated from different platforms used by real time network operating systems.

Algorithmic descriptions and representations included in this description are understood to be implemented by computer programs. Furthermore, it has also proven convenient at times, to refer to these arrangements of operations as modules or code devices, without loss of generality.

Unless otherwise indicated, discussions utilizing terms such as "selecting" or "computing" or "determining" or the like refer to the action and processes of a computer system, or similar electronic computing device, that manipulates and transforms data represented as physical (electronic) quantities within the computer system memories or registers or other such information storage, transmission or display devices.

The algorithms and displays presented are not inherently related to any particular computer or other apparatus. Various general-purpose systems may also be used with programs in accordance with the teachings above, or it may prove convenient to construct more specialized apparatus to perform the required method steps. The required structure for a variety of these systems will appear from the description above. In addition, a variety of programming languages may be used to implement the teachings above.

The description of the present invention is intended to be illustrative, but not limiting, of the scope of the invention as defined by the claims.

**Claims**

1. A computer implemented method comprising:

   accessing (610) a genotype dataset associated with an individual;
   dividing (620) the genotype dataset into a plurality of windows, each window comprising a set of single nucleotide polymorphisms (SNPs);
   determining (630) a pair of phased haplotype datasets from the plurality of windows of genotype dataset;
   determining (640), using a convolutional neural network (CNN), a plurality of emission probabilities for at least a particular window of a hidden Markov model (HMM), wherein the emission probabilities in the particular window are determined by the CNN that takes the pair of phased haplotype datasets as input to determine the probability of observing the pair of phased haplotype datasets within the particular window of the hidden Markov model given a pair of ethnicity labels;
   generating (650) the HMM that comprises a plurality of node groups and a plurality of edges, wherein the node group corresponding to the particular window comprises a plurality of nodes and each node is associated with one of the emission probabilities; and
   generating (660) information on ethnic origin of the individual using the HMM.

2. The computer implemented method of claim 1, further comprising:
   determining a second plurality of emission probabilities for a second window, wherein the second plurality of emission probabilities are determined by a second CNN that is different from the CNN.

3. The computer implemented method of claim 1, wherein each haplotype dataset is encoded as ordered binary values including a first value and a second value, the first value representing major allele and the second value representing minor allele.

4. The computer implemented method of claim 1, wherein the CNN comprises one or more convolutional layers, one or more pooling layers, and a fully connected layer.

5. The computer implemented method of claim 4, wherein the convolutional layer is configured to perform one-dimensional convolution, wherein the one-dimensional convolution uses a sliding window that moves in a direction along one of the phased haplotype datasets.

6. The computer implemented method of claim 1, wherein the CNN is trained using datasets of reference panels, wherein a reference panel includes a genotype dataset of an individual who is known to have an ethnicity label.

7. The computer implemented method of claim 6, wherein the CNN is trained with additional training samples, the additional training samples generated by combining segments from genotype datasets of individuals from the reference panels.

8. The computer implemented method of claim 1, wherein the CNN is trained by reducing errors in predicting the ethnicity labels.

9. A non-transitory computer readable storage medium storing instructions which, when executed by one or more processors, cause the one or more processors to perform a method according to any one of the preceding claims.

10. A system arranged to carry out a method according to any one of claims 1 to 8.

**Patentansprüche**

1. Computerimplementiertes Verfahren, umfassend:

   Zugreifen (610) auf einen Genotyp-Datensatz, der mit einem Individuum assoziiert ist;
   Aufteilen (620) des Genotyp-Datensatzes in eine Vielzahl von Fenstern, wobei jedes Fenster einen Satz von Einzelnukleotidpolymorphismen (single nucleotide polymorphisms; SNPs) umfasst;

Bestimmen (630) eines Paares von gephasten Haplotyp-Datensätzen aus der Vielzahl von Fenstern des Genotyp-Datensatzes;

Bestimmen (640), unter Verwendung eines faltungsneuronalen Netzwerks (convolutional neural network; CNN), einer Vielzahl von Emissionswahrscheinlichkeiten für mindestens ein bestimmtes Fenster eines verborgenen Markov-Modells (hidden Markov model; HMM), wobei die Emissionswahrscheinlichkeiten in dem bestimmten Fenster durch das CNN bestimmt werden, das das Paar gephaster Haplotyp-Datensätze als Input zur Bestimmung der Wahrscheinlichkeit der Beobachtung des Paares gephaster Haplotyp-Datensätze innerhalb des bestimmten Fensters des verborgenen Markov-Modells unter Berücksichtigung eines Paares von Ethnizitätskennzeichnungen verwendet;

Erzeugen (650) des HMM, das eine Vielzahl von Knotengruppen und eine Vielzahl von Kanten umfasst, wobei die dem bestimmten Fenster entsprechende Knotengruppe eine Vielzahl von Knoten umfasst und jeder Knoten mit einer der Emissionswahrscheinlichkeiten assoziiert ist; und

Erzeugen (660) von Informationen über die ethnische Herkunft des Individuums unter Verwendung des HMM.

2. Computerimplementiertes Verfahren nach Anspruch 1, ferner umfassend:
Bestimmen einer zweiten Vielzahl von Emissionswahrscheinlichkeiten für ein zweites Fenster, wobei die zweite Vielzahl von Emissionswahrscheinlichkeiten durch ein zweites CNN bestimmt wird, das von dem CNN verschieden ist.

3. Computerimplementiertes Verfahren nach Anspruch 1, wobei jeder Haplotyp-Datensatz als geordnete Binärwerte codiert ist, die einen ersten Wert und einen zweiten Wert umfassen, wobei der erste Wert das Hauptallel und der zweite Wert das Nebenallel darstellt.

4. Computerimplementiertes Verfahren nach Anspruch 1, wobei das CNN eine oder mehrere Faltungsschichten, eine oder mehrere Pooling-Schichten und eine vollständig verbundene Schicht umfasst.

5. Computerimplementiertes Verfahren nach Anspruch 4, wobei die Faltungsschicht so konfiguriert oder ausgebildet ist, dass sie eine eindimensionale Faltung durchführt, wobei die eindimensionale Faltung ein Gleitfenster verwendet, das sich in einer Richtung entlang eines der gephasten Haplotyp-Datensätze bewegt.

6. Computerimplementiertes Verfahren nach Anspruch 1, wobei das CNN unter Verwendung von Datensätzen von Referenzpanels trainiert wird, wobei ein Referenzpanel einen Genotyp-Datensatz eines Individuums umfasst, von dem bekannt ist, dass es eine Ethnizitätskennzeichnung hat.

7. Computerimplementiertes Verfahren nach Anspruch 6, wobei das CNN mit zusätzlichen Trainingsbeispielen trainiert ist oder wird, wobei die zusätzlichen Trainingsbeispiele durch Kombinieren von Segmenten aus Genotyp-Datensätzen von Individuen aus den Referenzpanels erzeugt werden.

8. Computerimplementiertes Verfahren nach Anspruch 1, wobei das CNN durch Reduzieren von Fehlern bei der Vorhersage der Ethnizitätskennzeichnungen trainiert ist oder wird.

9. Nicht-transitorisches, computerlesbares Speichermedium, das Anweisungen speichert, die, wenn sie von einem oder mehreren Prozessoren ausgeführt werden, den einen oder die mehreren Prozessoren veranlassen, ein Verfahren gemäß einem der vorstehenden Ansprüche durchzuführen.

10. System, das zum Ausführen eines Verfahrens gemäß einem der Ansprüche 1 bis 8 ausgebildet oder eingerichtet ist.

**Revendications**

1. Procédé mis en œuvre par ordinateur, comprenant :

accéder (610) à un ensemble de données génotypiques associé à un individu ;
diviser (620) l'ensemble de données génotypiques en une pluralité de fenêtres, chaque fenêtre comprenant un ensemble de polymorphismes nucléotidiques simples (SNP) ;
déterminer (630) une paire d'ensembles de données d'haplotypes phasés à partir de la pluralité de fenêtres de l'ensemble de données génotypiques ;

déterminer (640), à l'aide d'un réseau neuronal convolutif (CNN), une pluralité de probabilités d'émission pour au moins une fenêtre particulière d'un modèle de Markov caché (HMM), dans lequel les probabilités d'émission dans la fenêtre particulière sont déterminées par le CNN qui prend la paire d'ensembles de données d'haplotypes phasés en entrée pour déterminer la probabilité d'observer la paire d'ensembles de données d'haplotypes phasés dans la fenêtre particulière du modèle de Markov caché étant donné une paire d'étiquettes d'ethnicité ; générer (650) le HMM qui comprend une pluralité de groupes de nœuds et une pluralité d'arêtes, dans lequel le groupe de nœuds correspondant à la fenêtre particulière comprend une pluralité de nœuds et chaque nœud est associé à l'une des probabilités d'émission ; et générer (660) des informations sur l'origine ethnique de l'individu à l'aide du HMM.

2. Procédé mis en œuvre par ordinateur selon la revendication 1, comprenant en outre :
la détermination d'une deuxième pluralité de probabilités d'émission pour une deuxième fenêtre, dans laquelle la deuxième pluralité de probabilités d'émission est déterminée par un deuxième CNN qui est différent du le CNN.

3. Procédé mis en œuvre par ordinateur selon la revendication 1, dans lequel chaque ensemble de données d'haplotypes est codé sous forme de valeurs binaires ordonnées comprenant une première valeur et une deuxième valeur, la première valeur représentant l'allèle majeur et la deuxième valeur représentant l'allèle mineur.

4. Procédé mis en œuvre par ordinateur selon la revendication 1, dans lequel le CNN comprend une ou plusieurs couches convolutives, une ou plusieurs couches de regroupement et une couche entièrement connectée.

5. Procédé mis en œuvre par ordinateur selon la revendication 4, dans lequel la couche convolutive est configurée pour effectuer une convolution unidimensionnelle, dans lequel la convolution unidimensionnelle utilise une fenêtre coulissante qui se déplace dans une direction le long de l'un des ensembles de données d'haplotypes phasés.

6. Procédé mis en œuvre par ordinateur selon la revendication 1, dans lequel le CNN est entraîné à l'aide d'ensembles de données de panels de référence, un panel de référence comprenant un ensemble de données génotypiques d'un individu dont l'appartenance ethnique est connue.

7. Procédé mis en œuvre par ordinateur selon la revendication 6, dans lequel le CNN est entraîné à l'aide d'échantillons d'entraînement supplémentaires, les échantillons d'entraînement supplémentaires étant générés en combinant des segments provenant d'ensembles de données génotypiques d'individus issus des panels de référence.

8. Procédé mis en œuvre par ordinateur selon la revendication 1, dans lequel le CNN est entraîné en réduisant les erreurs dans la prédiction des étiquettes d'origine ethnique.

9. Support de stockage non transitoire lisible par ordinateur stockant des instructions qui, lorsqu'elles sont exécutées par un ou plusieurs processeurs, amènent le ou les processeurs à exécuter un procédé selon l'une quelconque des revendications précédentes.

10. Système agencé pour mettre en œuvre un procédé selon l'une quelconque des revendications 1 à 8.

**FIG. 1**

Computing Server
130

Genealogy Data
Store
200

Genetic Data
Store
205

Individual Profile
Store
210

Sample
Pre-Processing
Engine
215

Phasing
Engine
220

IBD Estimation
Engine
225

Community
Assignment Engine
230

IBD Network
Data Store
235

Reference Panel
Sample Store
240

Ethnicity
Estimation
Engine
245

Front-End
Interface
250

*FIG. 2A*

**Ethnicity Estimation Engine** <u>245</u>

Genealogy Data Store 252

Haploid MM Store 254

Reference Panel Sample Store 256

Diploid HMM Store 258

CNN Store 260

Annotation Store 262

Genetic Data Store 264

Inter-Window HMM Store 266

Labeling Model 290

Haploid MM module 268

Diploid HMM module 270

Inter-Window HMM Module 272

CNN Module 274

Label Assignment Module 276

Phasing Module 278

Range Module 280

Confidence Module 282

Polygon Module 284

**FIG. 2B**

EP 4 091 172 B1

## FIG. 2C

Reference panel for non-admixed population A {  ind1  ind2  ind3

Reference panel for admixed population B {  ind1  ind2  ind3  } Synthetic Genetic Dataset

Haploid Markov Model for Window $w$
300

FIG. 3A

number of states $h = 1$ | number of states $h = 2$ | number of states $h = 3$ | number of states $h = 5$ | number of states $h = 1$

Diploid HMM for Window $w$
301

FIG. 3B

Inter-Window HMM
400

*FIG. 4*

Boundary of
Chromosome

Window 1    Window 2    Window 3    Window *w*    Window *w*+1

Start
State
410

Inter-
chromosome
State
420

A node
group

EP 4 091 172 B1

*FIG. 5*

500

Access a genotype dataset associated with an
individual
610

Divide the genotype dataset into a plurality of
windows, each window comprising a set of SNPs
620

Determine a pair of phased haplotype datasets from
the plurality of windows
630

Determine emission probabilities for a particular
window by using a CNN
640

Generate a directed acyclic graph that comprises
nodes and edges, wherein each node is associated
with an emission probability
650

Generate information on ethnic origin of the
individual using the directed acyclic graph
660

FIG. 6

*FIG. 7*

## FIG. 8

```
┌─────────────────────┐
│ Receive Haplotype   │
│       Data          │
│       810           │
└─────────────────────┘
           │
           ▼
┌─────────────────────┐   ┌─────────────────────┐
│   Build haploid     │   │ Receive Reference   │
│  Markov Models      │   │ Panel Samples of    │
│       820           │   │    Label k          │
│                     │   │      Rₖ             │
│                     │   │      830            │
└─────────────────────┘   └─────────────────────┘
           │                         │
           └───────────┬─────────────┘
                       ▼
           ┌─────────────────────┐
           │    Calculate        │
           │   Annotations       │
           │       840           │
           └─────────────────────┘
                       │
                       ▼
           ┌─────────────────────┐
           │    Calculate        │
           │ Annotation Products │
           │       850           │
           └─────────────────────┘
                       │
                       ▼
           ┌─────────────────────┐
           │ Calculate Label Pair│
           │    Probability      │
           │   Distributions     │
           │       860           │
           └─────────────────────┘
                       │
                       ▼
           ┌─────────────────────┐
           │ Calculate Emission  │
           │    Probability      │
           │       870           │
           └─────────────────────┘
```

# FIG. 9

Receive an input sample
genotype dataset X
910

Phase input sample genotype
X to genetate a pair of
phased haplotype datasets
920

Build Inter-Window HMM with
(p, q, z) as hidden states
930

Initialize tau_m, tau_f, and
tau_z
940

Calculate transition
probabilities
950

# FIG. 10

```
┌─────────────────────┐      ┌─────────────────────┐
│ Calculate transition│      │ Calculate emission  │
│   probabilities     │      │   probabilities     │
│       1010          │      │       1020          │
└─────────────────────┘      └─────────────────────┘
```

```
┌─────────────────────┐
│  Compute Inter-     │◄─┐
│  window HMM         │  │
│       1030          │  │
└─────────────────────┘  │   Repeat
         │               │
         ▼               │
┌─────────────────────┐  │
│   Use Viterbi       │  │
│   Algorithm to      │  │
│ estimate tau_m,     ├──┘
│  tau_f, tau_z       │
│       1040          │
└─────────────────────┘
         │
         ▼
┌─────────────────────┐
│   Use Viterbi       │
│   Algorithm to      │
│ determine p, q, z   │
│       1050          │
└─────────────────────┘
```

Access a genotype dataset
associated with an individual
1110

Divide the genotype dataset into a
plurality of windows
1120

Determine a pair of phased
haplotype datasets from the
plurality of windows of the
genotype dataset
1130

Build, using the pair of phased
haplotype datasets, a hidden
Markov model
1140

Provide information of ethnic
origin of the individual using the
hidden Markov model
1150

*FIG. 11*

## *FIG. 12*

Identify a plurality of admixed individuals based on genealogical
data stored in an online system
1210

Retrieve Genetic Datasets of the plurality of identified admixed
individuals
1220

Identify a plurality of genetic segments that are inherited from an
ethnic origin from the retrieved genetic datasets
1230

Create a synthetic genetic dataset from a combination of the
plurality of identified genetic segments that are inherited from the
target ethnic origin
1240

Add the synthetic genetic dataset to a collection of reference panel
datasets as one of the reference panel samples
1250

# FIG. 13

Access a genotype dataset associated with a target admixed individual
1310

Divide the genotype dataset into a plurality of windows
1320

Determine a pair of phased haplotype datasets from the genotype dataset
1330

Generate data representing a directed acyclic graph
1340

Determine, for each label, a probability of having the label given the window of SNP sites in the one of the pair of phased haplotype datasets
1350

Determine a path traversing the directed acyclic graph
1360

Generate information of a genetic composition of the target admixed individual having the target ethnic origin by determining a number of nodes being labeled with the target ethnic origin and included in the path
1370

# FIG. 14

Genotype Data
1410

Label Assignment
Module
276

Ethnicity
Estimates
1420

Range Module
280

Confidence Module
282

Ethnicity
Ranges
1430

Ethnicity
Confidences
1440

*FIG. 15*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 93100920
- US 56795719
- US 62729840
- US 62743448 B
- US 62752523 B
- US 62858820 B
- US 62962786
- US 20190114219 A1
- US 51909915
- US 02976513
- US 51910417
- US 16801116
- US 20945816
- US 59109915
- US 20170262577
- US 20200082903 A1
- US 20170017752 A1
- US 20160350479
- WO 2016061568 A1